# EUROPEAN PATENT APPLICATION

(11) **EP 1 016 719 A1**
(43) Date of publication of application: **05.07.2000**
(21) Application number: 98941674.8
(22) Date of filing: 02.09.1998
(51) Int. Cl.: C12N 15/12, C12N 5/10, C12Q 1/02

(54) **DNA PARTICIPATING IN REGULATION OF EXPRESSION OF A PEBP2 ALPHA A GENE, AND USE THEREOF**

(30) Priority: 02.09.1997 JP 25425097; 15.10.1997 JP 29940797; 08.04.1998 JP 11413598
(71) Applicant: Sumitomo Pharmaceuticals Company, Limited, Osaka 541-8510 (JP)
(72) Inventor: HARADA, Hideyuki, Nishinomiya-shi, Hyogo 663-8154 (JP); FUJIWARA, Masanori, Ashiya-shi, Hyogo 659-0015 (JP); TAGASHIRA, Shuzo, Toyonaka-shi, Osaka 561-0802 (JP); OGAWA, Shinji, Nishinomiya-shi, Hyogo 662-0831 (JP); KATSUMATA, Takashi, Sanda-shi, Hyogo 669-1323 (JP); NAKATSUKA, Masashi, Mishima-gun, Osaka 618-0001 (JP)
(74) Representative: VOSSIUS & PARTNER
(86) International application number: JP9803920
(87) International publication number: WO9911787

(57) **Abstract**

A DNA participating in the regulation of expression of a PEBP2 αA gene; a recombinant plasmid comprising a combination of the DNA with a reporter gene; cells transformed with the plasmid; a method for screening an expression regulator for PEBP2 αA gene with the transformed cells; and an expression regulator for a PEBP2 αA gene obtained by the screening method.

## Description

### TECHNICAL FIELD

The present invention relates to a DNA which participates in the regulation of expression of PEBP2αA gene and to the use thereof. More particularly, it relates to a DNA which participates in the regulation of expression of PEBP2αA gene, to a recombinant plasmid in which the DNA is ligated to a reporter gene, to a transformed cell which has been transfected with the plasmid, to a method for screening regulators for PEBP2αA gene expression using the transformed cell, and to regulators for PEBP2αA gene expression identified by the screening method.

Among the regulators for PEBP2αA gene expression, enhancers for PEBP2αA gene expression are believed to increase amount of PEBP2αA gene expression, whereby osteogenesis is promoted through enhancement of PEBP2αA expression and, therefore, they are useful as an osteogenesis accelerator.

### BACKGROUND ART

A polyoma enhancer binding protein 2αA (PEBP2αA) is also called a core binding factor (CBF) A1, and it is one of transcription factors belonging to a gene family having a runt domain. Up to now, PEBP2αA has been thought to play some roles in the transcriptional regulation of T lymphocytes specific genes (Mol. Cell. Biol. 15, 1662-1670(1995)). Recently, however, it has been found that, in knockout mice for this PEBP2αA gene, the ability of bone formation is completely deficient and, as a reason therefor, inhibition of both differentiation and maturation of osteoblast has been found to occur. Thus, it has been found for the first time that PEBP2αA which had been suggested to have roles only in T lymphocytes plays an important role in osteogenesis (Cell, 89, 755-764, 765-77 (1997)).

It has been also found that undifferentiated osteoblastic cell lines or skin fibroblasts are differentiated into a lineage of osteoblasts when PEBP2αA is forced to be expressed in those cells (Cell, 89, 747-754(1997)), and it has been further shown that antisense DNA of PEBP2αA gene suppresses the expression of PEBP2αA, whereby differentiation of osteoblasts is suppressed (Cell, 89, 747-754 (1997)). Moreover, it has been reported that cleidocranial dysplasia (CCD) which is one of human genetic diseases is caused by genetic mutations of the PEBP2αA (Cell, 89, 747-754, 1997; Nature Genet., 16, 307-310 (1997)).

As such, it has been clarified that PEBP2αA is an essential factor for osteogenesis (differentiation and maturation of osteoblast) and also that PEBP2αA actually has an ability to induce differentiation of undifferentiated mesenchymal cells into osteoblast linage. From those findings, it is believed that, for example, a substance which is able to enhance the expression of PEBP2αA gene or a substance which is able to enhance the function of PEBP2αA will result in promotion of osteogenesis through the effect(s) of PEBP2αA and, therefore, such substances will be effective therapeutic agents for bone diseases such as osteoporosis. Incidentally, at present, there is no promising therapeutic agent useful as an osteogenesis stimulator for therapy or prevention of osteoporosis and, accordingly, there has been a demand for the development of pharmacological agents based upon a new idea to enhance the effects of PEBP2αA.

In order to find out substances which enhance the expression and the function of PEBP2αA gene, it is necessary to construct a screening system which allows efficient selection of test substances by using PEBP2αA or a gene therefor. However, there are no reports concerning the construction of such a screening yet.

Especially, to constuct the screening system for substances which enhance the expression of PEBP2αA gene as mentioned above, it is at first necessary to identify the regulatory region for the expression of PEBP2αA gene, i.e., so-called promoter and enhancer/silencer region (it may be called as just "promoter region"). Thus, it is presumed that a substance which can enhance the expression of PEBP2αA gene will affect the regulatory region for the expression of PEBP2αA gene either directly or indirectly and, therefore, the screening system for enhancers of PEBP2αA gene expression mentioned above should be a system which permits detection of the effect of test substances on the regulatory region for the expression of PEBP2αA. Accordingly, in the construction of such a screening system, the identification of the regulatory region for the expression of PEBP2αA is essential.

However, the regulatory region for the expression of PEBP2αA gene has not been identified yet. Therefore, a screening system for finding out pharmacological agents which enhance the expression of PEBP2αA gene has not been constructed yet.

### Disclosure of the Invention

Objects of the present invention are to provide a regulatory region for the expression of PEBP2αA, which had not been identified, in other words, to provide a DNA which participates in the regulation of the expression; to provide a method for screening regulators for PEBP2αA gene expression using the DNA; and to provide regulators for PEBP2αA gene expression identified by the screening method.

Thus, objects of the present invention are to provide a DNA which participates in the regulation of expression of PEBP2αA gene; a recombinant plasmid in which the DNA is ligated to a reporter gene; cells which have been transformed with the plasmid; a method for screening regulators for PEBP2αA gene expression using the transformed cell; and regulators for PEBP2αA gene identified by the screening method. Among the regulators for PEBP2αA gene expression, enhancers for PEBP2αA gene expression are particularly effective as osteogenesis stimulators since it is believed to increase the amount of PEBP2αA gene expression resulting in the stimulation of osteogenesis.

Based upon the finding that PEBP2αA plays an important role in osteogenesis as mentioned above, the present inventors planned to identify a DNA which participates in the regulation of expression of PEBP2αA gene which has not been identified yet and to construct a screening system to identify regulators for PEBP2αA gene expression by using the DNA.

The cDNA sequence of the previously known as PEBP2αA has been registered in Genbank with the Accession Number; D14636. Although it has been determined that methionine at the position 1016 is a start site for the translation, the 5'-terminal (start site for the transcription) of the DNA has not been determined yet. Therefore, the present inventors at first planned to determine the 5'-terminal of the cDNA and based upon the determined 5'-terminal, the present inventors planned to determine the regulatory region for the expression of PEBP2αA.

To be more specific, determination of 5'-terminal was attempted by 5'-RACE method (Methods Enzymol., 218, 340-358 (1993)). Incidentally, the 5'-RACE method was developed as a method for supplementing the difficulty in obtaining full length of cDNA by a conventional screening of cDNA library and it is a method by which cDNA at 5'- or 3'-terminal is amplified and cloned by PCR. As an experimental result of the 5'-RACE method, clones having various lengths were obtained and, among them, there were many clones in which an elongation took place only up to 25 bp upstream from the start site of translation (position 1016 of the cDNA registered in Genbank), in other words, only up to the position 991 of the cDNA registered in Genbank. Although there has been no report at all that the 5'-terminal is located around this position 991, the present inventors thought of the possibility that there are new cDNAs having its 5'-terminal around position 991, thus, isolated a fragment covering positions 1-1015 (SEQ ID NO:2) which were upstream region of the position 1016 (a start site of translation) and part of the fragment covering positions 319-1015 by means of PCR, and investigated whether they had a promoter activity by examination of reporter gene activity in cells after ligating them with a reporter gene. As a result, it has been found for the first time that there is a promoter activity in the regions which have been reported as cDNA up to now. In other words, it was found that a promoter activity is expressed at least in the positions 319-1015 of the base sequence depicted in SEQ ID NO:2 (hereinafter, the PEBP2αA gene regulated by the promoter thus obtained may be sometimes called PEBP2αA (II) gene for the sake of convenience).

The present inventors have introduced a plasmid wherein a reporter gene is connected with the 3'-terminal of the DNA comprising the base sequence depicted in the above-mentioned SEQ ID NO:2 into C3H10T1/2 which is an undifferentiated mesenchymal cell, and then added BMP which is known as an osteogenetic factor (differentiation-inducing factor to osteoblast) to the cell. As a result, the above-mentioned promoter activity was enhanced by BMP. Thus, it has been found that, in the base sequence depicted in SEQ ID NO:2, not only the above-mentioned promoter region but also an enhancer region which enhances the promoter activity by responding to the BMP are present.

As such, it has now been found that a region which participates in the regulation of expression of PEBP2αA gene is present in the DNA having the base sequence depicted in SEQ ID NO:2.

Recently, in Cell, 89, 747-754 (1997), there has been reported novel PEBP2αA (referred to as Osf2/Cbfal) wherein the 5'-terminal is different from the known clone previously registered in Genbank as mentioned above and the 5'-terminal is longer than the known clone by 82 amino acids. In the article, only amino acid sequence is disclosed and a regulatory region for the expression, finding of which is the object of the present invention, is not disclosed. Therefore, the present inventors have prepared degenerate primers based upon the amino acid sequence and attempted to clone a DNA fragment which corresponds to an upstream region of this novel PEBP2αA gene by PCR, whereupon we have succeeded in cloning of a novel DNA (SEQ ID NO:1) comprising 1853 bp (provided that the first 35 bp is derived from an adaptor sequence) upstream from the start site of translation. Further, we have investigated whether the DNA has the promoter activity by ligating a reporter gene to the region of positions 1-1811 or 1113-1832 of the DNA depicted in SEQ ID NO:1, whereupon the promoter activity has been found in both of them. This finding shows that the retention of at least the positions 1113-1811 among the newly-cloned DNA having the base sequence depicted in SEQ ID NO:1 is sufficient to exert the promoter activity (hereinafter, the PEBP2αA gene under the regulatory expression through the promoter thus obtained may sometimes be referred to as PEBP2αA (I) gene for the sake of convenience).

After that, the DNA having the base sequence depicted in SEQ ID NO:1, which was obtained by the above PCR, was used as a probe for isolating a genomic DNA clone containing the DNA. After getting genomic DNA clones spanned about 14 kb, the present inventors determined the base sequence and it (SEQ ID NO:4) was compared with that depicted in SEQ ID NO:1, whereupon it was found that the positions 4834-6651 of the base sequence depicted in SEQ ID NO:4 corresponded to that depicted in SEQ ID NO:1 (specifically, the positions 36-1853 of the base sequence depicted in SEQ ID NO:1). It was also found that the positions 4834-6651 of the base sequence of this SEQ ID NO:4 also exhibited promoter activity as the DNA having the base sequence depicted in SEQ ID NO:1. Incidentally, the positions 1113-1811 of the base sequence depicted in SEQ ID NO:1 as mentioned above, corresponds to the positions 5911-6609 of the base sequence depicted in SEQ ID NO:4.

Further, as a result of the detailed investigation on the promoter activity using the DNA having the base sequence depicted in SEQ ID NO:4, it was found that the region of the sequence positions 6491-6560 of SEQ ID NO:4 (the positions 1693-1762 of SEQ ID NO:1) was essential for the expression of the promoter activity.

The present inventors subsequently investigated the region which regulates the above-mentioned promoter activity, i.e. an enhancer/silencer region. The result was that:
1) a plasmid in which a reporter gene was ligated to 3'-terminal of the DNA comprising the positions 1-1811 of the base sequence depicted in SEQ ID NO:1 or of the DNA comprising the positions 4834-6651 of the base sequence depicted in SEQ ID NO:4, was introduced into C3H10T1/2 cells which is an undifferentiated mesenchymal cell and then BMP was added to the culture medium of the cells, whereby the above-mentioned promoter activity was enhanced by BMP. This shows that there was an enhancer region responding to BMP and enhancing promoter activity in the base sequence depicted in SEQ ID NO:1 and in the region of the positions 4834-6651 of the base sequence depicted in SEQ ID NO:4; and
2) in the sequence of SEQ ID NO:1 (positions 4834-6651 of SEQ ID NO:4), there were many binding sites for PEBP2 family including PEBP2αA itself and similar sequences thereto; binding sites for Sry family belonging to HMG family in which SOX9, an important factor for differentiation of chondrocytes and maintenance of characteristics of chondrocytes is belonging to, and similar sequences thereto; and binding sites for GATA family which is a transcription factor whose expression is regulated by BMP, an osteogenetic factor, and which is a factor supposed to have some roles in the function of BMP and similar sequences thereto (cf. Table 1 which will be mentioned later) and, when those transcription factors such as PEBP2, Sry and GATA were actually overexpressed in the cells with the reporter vector having the base sequence depicted in SEQ ID NO:1 (positions 4834-6651 of SEQ ID NO:4), the above-mentioned promoter activity was regulated. These results showed that, in the base sequence depicted in SEQ ID NO:1 (positions 4834-6651 of SEQ ID NO:4), there was a region which responds to those transcription factors and regulates promoter activity.

Incidentally, in connection with the fact that the binding sites for the specific transcription factors mentioned above was an enhancer/silencer region participating in tissue-specific gene expression, the following reports are available.

Thus, for example, the binding sites for PEBP2 family are also present in the regulatory region for the expression of osteopontin and osteocalcin genes which are differentiation markers of osteoblasts, and it is known that, as a result of the binding of PEBP2αA to the binding site, gene expressions of osteopontin and osteocalcin are stimulated (Cell, 89, 747-754 (1997)). It is also known that a binding site for SOX is existed in an expression regulatory region (intron) of the type 2 collagen, which is known as a differentiating marker of chondrocytes and thus this element plays an important role for a tissue-specific gene expression (Nature Genet., 15, 21-29 (1997)). A GATA binding site was found to exist in a promoter region of human BMP5 which is supposed to play an important role in the formation of bone and cartilage and in the process of healing of bone fracture. This shows that the binding sequence may possibly regulate the gene expression of BMP5 (Biochem. Biophys. Res. Commun., 221, 768-772 (1996)). Accordingly, it is believed that, when a transcription factor bound to all or part of the binding sites for transcription factor family as shown in Table 1, PEBP2αA (I) gene expression, which is specific in bone and cartilage, is regulated.

As such, it was found that there are some regions participating in the regulation of the expression of PEBP2αA gene in the base sequence depicted in SEQ ID NO:1 or in the positions 4834-6651 of the base sequence depicted in SEQ ID NO:4.

Further, it was recently pointed out that the N terminal of PEBP2αA (I), i.e. the start site for translation, is not the first methionine (position 6652 of the base sequence depicted in SEQ ID NO:4) disclosed as the N-terminal in the above-mentioned article, Cell, 89, 747-754 (1997) but may possibly be the second methionine (position 7053 of the base sequence in SEQ ID NO:4) (Proc. Natl. Acad. Sci. USA, 94, 8646-8651 (1997); Jikken Igaku, 16(2), 107-113 (1998)). Hereinafter, the above-mentioned PEBP2αA (I) wherein the first methionine is assigned as N terminal may be sometimes called PEBP2αA (I)-L, while PEBP2αA (I) having the second methionine as N terminal may be called PEBP2αA(I)-S in order to distinguish them in some situation. The present inventors have supposed that, for the regulation of expression of PEBP2αA(I)-S, not only the region upstream from the first methionine, which was already determined to be an expression regulatory region (the base sequence depicted in SEQ ID NO:1 and positions 4834-6651 of the base sequence depicted in SEQ ID NO:4), but also the region between the first and the second methionine (positions 6652-7052 of the base sequence depicted in SEQ ID NO:4) may be participated in.

Accordingly, by analyzing the sequence between the first and the second methionine, it was found that there existed the above-mentioned binding sites for PEBP2 family, binding sites for Sry family, binding sites for GATA family and many similar sequences thereto in this region (cf. Table 1). When those transcription factors such as PEBP2, Sry and GATA were actually applied to the luciferase vector containing the DNA of the positions 4834-7052 of SEQ ID NO:4, the above-mentioned promoter activity was regulated. In addition, it was also found that the sequence between the first and the second methionine actually regulated the promoter activity. Such results have revealed that there is a region participating in the regulation of expression of PEBP2αA (I) gene not only in the positions 4834-6651 of the base sequence of SEQ ID NO:4 but also in the positions 6652-7052 of the base sequence of SEQ ID NO:4.

By constructing a plasmid in which the DNA participating in the expression regulation of the PEBP2αA (I) gene obtained above or the DNA participating in the expression regulation of PEBP2αA (II) is ligated to a reporter gene such as luciferase or CAT (in the case where only an enhancer/silencer region of the DNA participating in the expression regulation of PEBP2αA gene is used, a plasmid which further contains a promoter should be constructed), introducing the plasmid into cells, adding a test substance to the cells, and detecting whether the test substance increases the transcription of the reporter gene, enhancers for PEBP2αA gene expression can be easily identified. Since PEBP2αA is an essential factor for osteogenesis as previously mentioned in the paragraph of "Prior Art", it is believed that the expression enhancers for PEBP2αA promote osteogenesis by increasing the amonunt of expressed PEBP2αA. Incidentally, as previously mentioned, an increase in the transcription of reporter gene was observed by affection of BMP which is an osteogenetic factor. Such a result shows that the present screening system is an actually useful system and that expression enhancers of PEBP2αA selected by the present screening system may have an osteogenesis promoting effect.

Further, the screening system of the present invention is useful not only for finding out expression enhancers for PEBP2αA gene but also for expression suppressors. The expression suppressors for PEBP2αA gene found by means of the present screening system can be a preventive or therapeutic agent for, for example, ectopic ossification and ectopic calcification.

Thus, the present invention has been accomplished based upon the above-mentioned findings.

Thus, the characteristic features of the present invention are:
(1) A DNA which comprises all or part of the base sequence depicted in any one of SEQ ID NO:1, SEQ ID NO:2 or SEQ ID NO:4 and participates in the regulation of expression of PEBP2αA gene.
(2) The DNA according to (1), wherein the DNA comprises all or part of the sequence of positions 4834-7052 in the base sequence depicted in SEQ ID NO:4.
(3) The DNA according to (1) or (2), wherein the DNA comprises the DNA selected from the following (a) to (c):
(a) a DNA which comprises at least the sequence at positions 1693-1762 of the base sequence depicted in SEQ ID NO:1 or the corresponding sequence at positions 6491-6560 of the base sequence depicted in SEQ ID NO:4;
(b) a DNA which comprises at least the sequence at positions 1113-1811 of the base sequence depicted in SEQ ID NO:1 or the corresponding sequence at positions 5911-6609 of the base sequence depicted in SEQ ID NO:4; and
(c) a DNA which comprises at least the sequence at positions 319-1015 of the base sequence depicted in SEQ ID NO:2.

(4) The DNA according to (1) or (2), wherein the DNA comprises any one of the binding sequences for PEBP2 family, the binding sequences for Sry family, the binding sequences for GATA family or sequences similar to those binding sequences, as listed in the following Table 1:

**Table 1**

| Positions in SEQ ID NO:4 | Positions in SEQ ID NO:1 |
|---|---|
| | |

| . Binding sequence for PEBP2 Family or Similar Sequence Thereto | |
|---|---|
| positions 5627-5633 | positions 829-835 |
| positions 6307-6313 | positions 1509-1515 |
| positions 6571-6577 | positions 1773-1779 |
| positions 6674-6680 | - |
| positions 6682-6688 | - |
| positions 6801-6807 | - |

| . Binding sequence for Sry Family or Similar Sequence Thereto | |
|---|---|
| positions 5069-5075 | positions 271-277 |
| positions 5424-5435 | positions 626-637 |
| positions 5600-5606 | positions 802-808 |
| positions 5644-5650 | positions 846-852 |
| positions 5756-5762 | positions 958-964 |
| positions 5925-5931 | positions 1127-1133 |
| positions 5936-5942 | positions 1138-1144 |
| positions 6630-6636 | positions 1832-1838 |
| positions 6670-6676 | - |
| positions 6853-6859 | - |
| positions 6858-6864 | - |

| . Binding sequence for GATA Family or Similar Sequence Thereto | |
|---|---|
| positions 5014-5023 | positions 216-225 |
| positions 5050-5059 | positions 252-261 |
| positions 5093-5106 | positions 295-308 |
| positions 5162-5171 | positions 364-373 |
| positions 5288-5297 | positions 490-499 |
| positions 5578-5588 | positions 780-790 |
| positions 5676-5685 | positions 878-887 |
| positions 5790-5799 | positions 992-1001 |
| positions 5836-5845 | positions 1038-1047 |
| positions 6484-6492 | positions 1686-1694 |
| positions 6576-6585 | positions 1778-1787 |
| positions 7024-7036 | - |

(5) A DNA which hybridizes under stringent conditions with the DNA described in any one of (1)-(4), or a DNA which contains deletion, substitution and/or addition of one or more bases in the DNA described in any one of (1)-(4), said DNA participating in the regulation of expression of PEBP2αA gene.
(6) The DNA according to (5), wherein the DNA comprises all or part of the base sequence depicted in SEQ ID NO:5.
(7) A plasmid in which a reporter gene is ligated to the DNA described in any one of (1)-(6).
(8) A plasmid in which a promoter gene and a reporter gene are ligated to the DNA described in any one of (1)-(6).
(9) The plasmid according to (7) or (8), wherein the reporter gene is a luciferase gene.
(10) A transformed cell obtained by introducing the plasmid described in any one of (7) to (9).
(11) A method for screening expression regulators for PEBP2αA gene, characterized by adding a substance to be tested to the transformed cell described in (10) and detecting whether the substance affects the DNA described in any one of (1)-(6).
(12) An expression regulator for PEBP2αA gene which is obtained through the screening method described in (11).
(13) An expression enhancer for PEBP2αA gene which is obtained through the screening method described in (11).

To be specific, "PEBP2αA gene" in the present invention means the above-mentioned PEBP2αA (I) gene or PEBP2αA (II) gene. It is believed that, in PEBP2αA (I) gene, there are two types, one being PEBP2αA (I)-L which is longer than PEBP2αA (II) by 82 amino acids and the other being PEBP2αA (I)-S which is longer than PEBP2αA (II) by 14 amino acids (cf. Jikken Igaku, 16, 107-113 (1998)).

The term "DNA participating in the regulation of expression" in the present invention stands for "a region for the regulation of expression" of a gene and, to be more specific, it means DNA which participates in the regulation of expression of the gene and which is called promoter, enhancer and silencer . Incidentally, "promoter and enhancer/silencer" is sometimes called just "promoter". However, in the present specification, "promoter" stands for a DNA region which is necessary for recognition and binding by RNA polymerase, while "enhancer" stands for a DNA region which regulates the above-mentioned promoter activity (transcriptional activity).

Specific examples of the DNA which participates in the regulation of expression of PEBP2αA gene are as follows.

Thus, in the case of PEBP2αA (I) gene, a DNA which comprises all or part of the base sequence depicted in SEQ ID NO:1 or SEQ ID NO:4 and participates in the regulation of expression of PEBP2αA (I) gene, is included in the scope of the present invention. In the case of PEBP2αA (II) gene, a DNA which comprises all or part of the base sequence depicted in SEQ ID NO:2 and participates in the regulation of expression of PEBP2αA (II) gene, is included in the scope of the present invention.

The DNA having a base sequence depicted in SEQ ID NO:1 and SEQ ID NO:4 contains an expression regulatory region for Osf2/Cbfal reported in Cell, 89, 747-754 (1997) (which is called PEBP2αA (I) or PEBP2αA (I)-L according to the present invention) and an expression regulatory region for Osf2/osteoblast-specific Pebp2αA A1 (Cbfal) having the second methionine as N-terminal, which was reported in Jikken Igaku, 16(2), 107-113 (1998) (which is called PEBP2αA (I)-S according to the present invention). The base sequence depicted in SEQ ID NO:4 contains the base sequence depicted in SEQ ID NO:1 and it is a genome sequence having a length of about 14 kb. The sequence at positions 4834-6651 of the base sequence depicted in SEQ ID NO:4 corresponds to the base sequence depicted in SEQ ID NO:1 (incidentally, the first 35 bp of the base sequence depicted in SEQ ID NO:1 is a sequence derived from an adaptor sequence).

The DNA can be cloned by the method described in Example 1 or Example 6 as mentioned hereinafter. However, as the result of laid-open of the base sequences depicted in SEQ ID NO:1 and SEQ ID NO:4, the DNA having the base sequence of SEQ ID NO:1 or SEQ ID NO:4 can be easily cloned by using all or part of the DNA as a probe or a PCR primer, while using, for example, a genomic library of mouse liver (Clontech). Further, the DNA having the base sequence depicted in SEQ ID NO:4 can be isolated by hybridization with the DNA depicted in SEQ ID NO:1 as a probe. The cloning can be easily carried out by those skilled in the art according to, for example, Molecular Cloning, 2nd Ed., Cold Spring Harbor Laboratory Press (1989). Furthermore, the DNA comprising part of the base sequence depicted in SEQ ID NO:1 or SEQ ID NO:4 can be cloned by the use of an appropriate part of the base sequence depicted in SEQ ID NO:1 or SEQ ID NO:4 as a PCR primer.

The promoter activity of thus-obtained DNA having all or part of the base sequence depicted in SEQ ID NO:1 or SEQ ID NO:4 can be measured by, for example, constructing a plasmid by inserting a candidate DNA for the above promoter at the 5'-terminal of luciferase gene in a luciferase vector having no promoter, such as pGL3-Basic Vector (Promega), introducing the plasmid into appropriate cells, culturing the cells, and subjecting the cells to cytolysis, followed by subjecting the cytolyzed liquid to, for example, luciferase assay reagent (Promega).

In addition, the enhancer/silencer activity of the above-mentioned DNA can be measured by the use of a luciferase vector into which a DNA functional as a promoter (SV40 promoter, etc.) has been already inserted at the 5'-terminal of a luciferase gene, such as pGL3-Promoter Vector (Promega).

Among the base sequences depicted in SEQ ID NO:4, a promoter activity or an enhancer/silencer activity was detected in various DNAs having all or part of DNAs of the positions 4834-7052. Accordingly, as a preferred embodiment of the DNA having all or part of the base sequence depicted in SEQ ID NO:4, a DNA which has all or part of the positions 4834-7052 of the base sequence depicted in SEQ ID NO:4 and participates in the regulation of expression of PEBP2αA gene may be mentioned.

Among the DNA participating in the regulation of expression of PEBP2αA (I) gene, it has been found already that, so far as the sequence having a promoter activity is concerned, the positions 1693-1762 of the base sequence depicted in SEQ ID NO:1 (the positions 6491-6560 of the base sequence depicted in SEQ ID NO:4) is an essential sequence for expression of a promoter activity and, therefore, a DNA which comprises at least this sequence will become a promoter region for PEBP2αA (I) gene.

It has been also found that a promoter activity is found in the DNA comprising the positions 1-1811 of the base sequence depicted in SEQ ID NO:1 (the positions 4834-6609 of the base sequence depicted in SEQ ID NO:4) or the positions 1113-1832 of the base sequence depicted in SEQ ID NO:1 (the positions 5911-6630 of the base sequence depicted in SEQ ID NO:4) and, therefore, as a preferred embodiment of the sequence having the above promoter activity, a DNA which comprises at least the positions 1113-1811 of the base sequence depicted in SEQ ID NO:1 (the positions 5911-6609 of the base sequence depicted in SEQ ID NO:4) may be exemplified.

Among the above, examples of far better ones in a sense that the promoter activity is strong, are a DNA comprising the positions 1(36)-1811 of the base sequence depicted in SEQ ID NO:1 (the positions 4834-6609 of the base sequence depicted in SEQ ID NO:4) and a DNA comprising the base sequence depicted in SEQ ID NO:1 (the positions 4834-6651 of the base sequence depicted in SEQ ID NO:4).

By investigating a reporter plasmid ligated to various mutants having deletion in the promoter region as mentioned above, the minimum portion having a promoter activity may be determined.

The sequence having an enhancer/silencer activity in the DNA participating in the regulation of expression of PEBP2αA (I) gene has been found to exert an enhancer/silencer activity to various DNAs having all or part of the positions 4834-6651 (SEQ ID NO:1) of the base sequence depicted in SEQ ID NO:4 and the positions 6652-7052 of the base sequence depicted in SEQ ID NO:4 and, therefore, a DNA having all or part of positions 4834-7052 of the base sequence depicted in SEQ ID NO:4 may be exemplified as an enhancer/silencer of the present invention.

Actual example is a base sequence depicted in SEQ ID NO:1 having an enhancer region capable of responding to BMP (the positions 4834-6651 of the base sequence depicted in SEQ ID NO:4). In addition, among the DNAs comprising part of the base sequence depicted in SEQ ID NO:1 or SEQ ID NO:4 for example, the DNA which has any one of the binding sequence for PEBP2 family, the binding sequence for Sry family, the binding sequence for GATA family and sequences similar thereto as listed in Table 1 and shows an enhancer/silencer activity to PEBP2αA (I) gene promoter may be exemplified as well.

Thus, it has been found that PEBP2αA belonging to a PEBP2 family is an essential factor for osteogenesis, that SOX9 belonging to Sry family is an important factor for differentiation of chondrocytes and maintenance of characteristics of chondrocytes, and that GATA family is a factor which partly plays a role for signal transduction of BMP (Cell, 89, 747-754 and 755-764 (1997); Nature Genet. 15, 21-29 (1997); Dev. Genet. 18, 267-278 (1996); Blood, 88, 1965-1972 (1996); Genes Dev. 11, 451-462 (1997)). As mentioned already, it is known that a binding sequence of such a factor represents a regulatory region participating in a tissue-specific gene expression (Cell, 89, 747-754; Nature Genet. 15, 21-29 (1997); Nature, 339, 448-451 (1989)). The present inventors have actually treated the luciferase vector containing the sequence of positions 4834-6651 in the base sequence depicted in SEQ ID NO:4, the luciferase vector containing the sequence of positions 4834-7052 in the base sequence depicted in SEQ ID NO:4, etc. with the above-mentioned transcription factors, whereupon the promoter activity of the PEBP2αA (I) gene has been regulated. Accordingly, it is apparent that the positions 4834-7052 of the base sequence depicted in SEQ ID NO:4 have an enhancer/silencer activity and, to be more specific, the DNA which has any one of the sequences listed in Table 1 and shows an enhancer/silencer activity to PEBP2αA (I) gene promoter may be exemplified as an enhancer/silencer of the present invention.

The present inventors have further found that various sequences which actually regulate the promoter activity are present in the sequence of positions 6652-7052 corresponding to the sequence between the first and second methionines in the base sequence depicted in SEQ ID NO:4. Accordingly, a DNA which has all or part of the positions 6652-7052 and shows an enhancer/silencer activity to PEBP2αA (I) gene promoter may be exemplified as an enhancer/silencer of the present invention.

Among the above, examples of an appropriate enhancer are the sequences of positions 6652-6665 and positions 6666-6703 in the base sequence depicted in SEQ ID NO:4, while examples of an appropriate silencer are the sequences of positions 6704-6782 and positions 6783-7052 in the base sequence depicted in SEQ ID NO:4.

Incidentally, a DNA having any one of the above-mentioned binding sequences will be an enhancer/silencer particularly participating in a bone- and cartilage-specific gene expression.

In the meanwhile, it is believed that PEBP2αA (I) gene is exclusively expressed in relatively differentiated osteoblast linage cells as shown in Examples 14 and 15 and, accordingly, it is believed that a DNA which has all or part of the base sequence depicted in SEQ ID NO:1 or SEQ ID NO:4 and participates in regulation of expression of PEBP2αA gene is an expression regulatory region which specifically affects the above-mentioned relatively differentiated osteoblasts. Therefore, for finding out osteogenesis enhancers, it is preferred to use those expression regulatory regions for PEBP2αA (I) gene.

The DNA having a base sequence depicted in SEQ ID NO:2 is located at the middle of the sequence registered in Genbank as a mouse PEBP2αA cDNA (Accession Number: D14636) and contains an expression regulatory region for PEBP2αA (which is called PEBP2αA (II) in the present specification). The DNA can be cloned by the method shown in Example 2 which will be described hereinafter. However, as a result of laid-open of the base sequence depicted in SEQ ID NO:2 to the public, a DNA having the base sequence depicted in SEQ ID NO:2 can be easily cloned when all or part of the DNA is utilized as a probe or a PCR primer and a genomic library from mouse liver (Clontech) is used, for example. The cloning can be easily carried out by those skilled in the art according to, for example, Molecular Cloning, 2nd Ed., Cold Spring Harbor Laboratory Press (1989). A DNA comprising part of the base sequence depicted in SEQ ID NO:2 can be cloned using an appropriate portion of the base sequence of SEQ ID NO:2 as a PCR primer as well.

With regard to the sequence having a promoter activity in the DNA participating in the regulation of expression of PEBP2αA (II) gene, it has been found that a promoter activity can be found in the DNA comprising the positions 1-1015 or positions 319-1015 of the DNA depicted in SEQ ID NO:2 and, therefore, any sequence comprising at least positions 319-1015 of the base sequence depicted in SEQ ID NO:2 can be a promoter region for PEBP2αA (II) gene.

Incidentally, with regard to a sequence having an enhancer/silencer activity in the DNA participating in the regulation of expression of PEBP2αA (II) gene, the sequence of positions 1-1015 of the base sequence depicted in SEQ ID NO:2 may be exemplified.

The minimum DNA sequence having a promoter activity may be determined by preparing and subjecting a reporter plasmid ligated to various mutants having deletion in the promoter region as previously mentioned to a test for transcriptional activity.

In addition, a DNA which is hybridized under a stringent condition with the DNA participating in the regulation of expression of PEBP2αA (I) gene and PEBP2αA (II) gene or a DNA which has deletion, substitution and/or addition of one or more bases in the DNA is fallen within the scope of the present invention so far as the DNA can participate in the regulation of expression of PEBP2αA gene, in other words, the DNA has a promoter activity or an enhancer/silencer activity. Specific examples are DNAs participating in the regulation of expression of PEBP2αA gene in all of vertebrate animals such as human being (SEQ ID NO:5) and rat.

"DNA which is hybridized under a stringent condition" used herein stands for a DNA which hybridizes with the above-mentioned DNA under, for example, a standard hybridizing condition (formamide concentration: 50%; salt concentration: 5 × SSC; temperature: 42°C). Such a DNA is cloned by means of hybridization, etc. with a DNA comprising, for example, part of the base sequence depicted, for example, in SEQ ID NO:1, SEQ ID NO:2 or SEQ ID NO:4. Actual cloning methods have been known and can be easily carried out by referring, for example, to Molecular Cloning, 2nd Ed., Cold Spring Harbor Laboratory Press (1989). An example of the genomic library used therein is a library of human placenta (Clontech).

A DNA which has deletion, substitution and/or addition of one or more bases in the above-mentioned DNA and participates in the regulation of expression of PEBP2αA gene stands for an artificially prepared and modified DNA which can participate in the regulation of expression of PEBP2αA gene, in other words, which has a promoter activity or an enhancer/silencer activity. The modified DNA can be easily prepared by those skilled in the art by means of a site-specific mutagenesis (Methods in Enzymology, 100, 468-(1983)), PCR (Molecular Cloning, 2nd Edt., Chapter 15, Cold Spring Harbor Laboratory Press (1989)), etc. The numbers of the DNAs which should be deleted, substituted, and/or added are the numbers which can be deleted, substituted, and/or added by known methods such as the above-mentioned site-specific mutagenesis.

The DNA which participates in the regulation of expression of PEBP2αA gene as mentioned above is utilized in a screening of an expression regulator for PEBP2αA gene which will be mentioned hereinafter. A method for the construction of the screening system, a method for the screening, etc. will be described below.

First, as a plasmid to be utilized for screening, a plasmid in which a reporter gene is ligated to the DNA participating in the regulation of expression of PEBP2αA gene is prepared. When only the enhancer/silencer region of PEBP2αA gene is used, it is necessary to insert any foreign promoter into in addition to the above-mentioned reporter gene.

As mentioned already, there is high possibility that PEBP2αA (I) gene is exclusively expressed in a relatively differentiated osteoblastic lenage cells and, therefore, it is preferred to use an expression regulatory region for PEBP2αA (I) gene for the purpose of screening, for example, enhancing agents for expression of PEBP2αA gene for the stimulation of osteogenesis. There is no particular limitation for the reporter gene to be used so far as it is a gene which can detect the enhancing activity for PEBP2αA gene expression. For example, a reporter vector in which a gene of luciferase, CAT (chloramphenicol acetyltransferase), ALP (alkaline phosphatase), GH (growth hormone) or the like has been integrated is commercially available, and any one of them can be used.

A foreign promoter to be used when only the enhancer or silencer region of PEBP2αA gene is used may be promoters of, for instance, SV40, β-globin, thymidine kinaze, etc. Promoter vectors into which such promoter and reporter genes mentioned above have been integrated are commercially available and they can be conveniently used in the present invention.

The plasmid prepared as such is introduced into cells to prepare a transformed cell. The transformed cell can be easily prepared by introducing the above-mentioned plasmid into the cell using a calcium phosphate method or LT-1 reagent(Panvera). There is no particular limitation for the cell to be used for the introduction so far as the cell permits detection of a transcription activity of reporter gene (promoter activity). However, for screening an expression enhancing agents for PEBP2αA gene for the stimulation of osteogenesis, which will be mentioned hereinafter, it is preferred to use an osteoblast-like cell line or an undifferentiated cell line having a property of being differentiated to osteoblasts. Further, the transformed cell may be the one which transiently expresses the reporter gene from the above-mentioned plasmid or the one which holds the gene in a stable manner (stable transformant). In particular, a stable transformant is a preferred cell because a troublesome operation for transfection of a gene for every screening can be omitted and the screening can be carried out more easily and within shorter time. The stable transformant can be prepared, for example, by introducing pSV2neo together with the above-mentioned plasmid, followed by selecting the resistant cells using antibiotic G418. Particularly preferred one among the cells prepared as such is a cell which exhibits high activity-increasing or -decreasing rate by a substance which increases or decreases the transcription activity (promoter activity). In other words, a cell by which the effects on the transcription activity can be easily detected is preferable.

Screening of an expression regulator for PEBP2αA gene can be carried out as follows using such a transformed cell. The screening method includes 1) a step where a substance to be tested is added to the above-mentioned transformed cell culture and 2) a step for detecting whether the substance regulates the promoter activity of the transformed cell. In the case of a high throughput screening which uses, for example, luciferase activity as an index, the following method is carried out. Thus, the above-mentioned stable transformed cells are plated in a 96-well culture plate so that 400,000 cells/100µl/well and incubation is carried out for 24 hours. With regard to a culture medium therefor, Eagle's Basal Medium (BME) (Gibco) to which 10% of fetal bovine serum has been added is used in the case of, for example, C3H10T1/2 which is an undifferentiated mesenchymal cell. 25µl of a solution of the substance to be tested (10µg/ml) is added to each of the wells (final concentration: 2µg/ml) and incubation is continued for 24 hours. After completion of the incubation, the supernatant is recovered by suction, a substrate for luciferase (LucLite; Packard) is added to the plate, and a quantitative determination is carried out by a conventional means. This allows the screening of the desired expression regulators for PEBP2αA gene.

When the above-mentioned substance to be tested is added to such a screening system, a substance which increases the promoter activity and a substance which suppresses the promoter activity can be selected. The substance which increases the promoter activity can be an expression enhancer of PEBP2αA gene, while the substance suppressing the promoter activity can be an expression suppressor for PEBP2αA gene.

As mentioned in the paragraph of "Prior Art", PEBP2αA is an essential factor for osteogenesis. Accordingly, it is believed that the above expression enhancer for PEBP2αA gene is able to promote osteogenesis by increasing the amount of expression of PEBP2αA and, therefore, the enhancer can be a therapeutic or preventive agent for bone diseases such as osteoporosis. As mentioned previoiusly, it has now been confirmed that, in the DNA comprising the base sequence depicted in SEQ ID NO:1, SEQ ID NO:2 and SEQ ID NO:4,there exists an enhancer region which enhances promoter activity through the effect of BMP which is an osteogenetic factor. Accordingly, it is believed that a proteinous factor, a low-molecular compound or the like acting on the enhancer region can be an expression enhancer for PEBP2αA like BMP and exhibits an osteogenesis promoting effect.

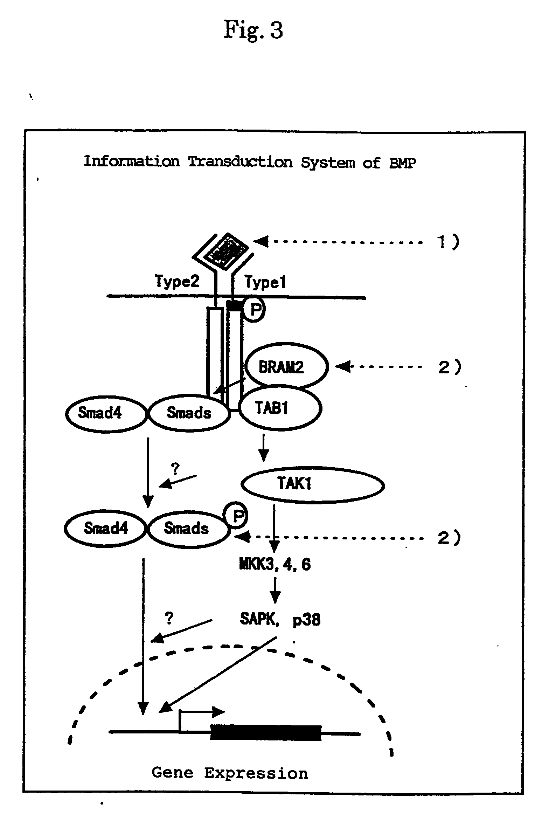

Examples of the substance affecting the enhancer region responding to BMP are 1) an agonist-like factor or agonist-like low-molecular compound acting on a BMP receptor [cf. 1) in Fig. 3] and 2) a factor or a low-molecular compound acting on signal transduction pathway of BMP [cf. 2) in Fig. 3].

As shown in 1) of Fig. 3, an agonist-like factor or agonist-like low-molecular compound acting on a BMP receptor as mentioned above, 1) is a proteinous factor or a low-molecular compound which binds to a receptor for BMP (Principles of Bone Biology, p.661-671, Academic Press, 1996; Bone, 19(6), 569-574 (1996)) as a ligand and is a substance which, as a result of binding to a BMP receptor instead of BMP, exerts the same signal transduction as BMP and, eventually, enhances the expression of PEBP2αA (increasing the promoter activity of PEBP2αA).

As shown in 2) of Fig. 3, a factor or a low-molecular substance affecting the signal transduction pathway of BMP, 2) is a proteinous factor (Nature, 390, 465-471 (1997); Genes Cells, 3, 257-264 (1998)) or a low-molecular compound acting on a factor such as BRAM or Smads which is so-called "signal-transducing factor". Thus, it stands for a proteinic factor or low-molecular compound by which, a gene expression of Smads or BRAM is regulated or a modification of these factors such as phosphorylation is carried out, the same effect as BMP is exerted during the course of signal transduction pathway of BMP and, finally, expression of PEBP2αA is enhanced. In addition, a mediating factor such as Smads and BRAM, a factor or a low-molecular compound capable of binding to an intracellular region of a BMP receptor, and a factor or a low-molecular compound which can promote the binding of the factor or low-molecular compound is fallen within the category of the above 2) provided that it eventually has an enhancing effect of expression of PEBP2αA.

Examples of the expression regulators for PEBP2αA gene besides the above-mentioned BMP-related substances are factors which can differentiate the undifferentiated mesenchymal cells such as TGFβs, IGFs and FGFs in addition to retinoic acid shown in the Examples; a growth factor which is believed to have an osteogenesis promoting effect; a PEBP2 family having its binding sequence in the base sequence depicted in SEQ ID NO:4; an Sry family including SOX9 which is an important factor for maintenance of characteristics of chondrocytes; and a GATA family. A factor or a low-molecular compound which interacts the binding sequence of those PEBP2 family, Sry family and GATA family can be an expression regulator of the present invention provided that it eventually has an expression regulatory effect on PEBP2αA.

On the other hand, an expression suppressor for PEBP2αA can be a pharmaceutical agent for prevention or therapy of ectopic ossification or ectopic calcification due to the following reasons.

Thus, there is a report (Saibo Kogaku, 13(12), 28-37 (1994)) that osteopontin which is a bone matrix protein is expressed in a calcified area in blood vessel and, since expression of PEBP2αA having a function of regulation of expression of bone matrix protein such as osteopontin is suggested with high possibility, it is presumed that PEBP2αA affects such ectopic ossification and ectopic calcification. Accordingly, it is believed that an expression suppressor which suppresses the expression of PEBP2αA in such cells (smooth muscle cells of blood vessels are exemplified in the case of calcification of blood vessels, while in the case of ectopic ossification, cells of ligaments or muscle are exemplified) will be a preventive or therapeutic agent for the above-mentioned ectopic ossification or ectopic calcification. Examples of such an expression suppressor for PEBP2αA gene are the factors which have been reported to actually suppress the calcification in smooth muscle cell of blood vessel such as bisphosphonate and PTHrP.

When such an expression regulator for PEBP2αA is used for a pharmaceutical composition, it can be used by a conventional means. For example, for oral administration, it can be administrated in a conventional dosage form such as tablets, capsules, syrup and suspension. In the case of parenteral administration, it can be administrated in the form of a liquid preparation such as solution, emulsion or suspension as an injection preparation, or in the form of suppository for rectum. Such a dosage form can be manufactured by a conventional method by mixing the active ingredient with usual carrier, filler, binder, stabilizer, etc. When used as an injective preparation, buffer, auxiliary solubilizer, isotonic agent, etc. may be added. Dose and administering frequency may vary depending upon the disease to be treated, symptom, age, body weight, etc. of a particular patient, dosage form, etc. In the case of an oral administration, the active ingredient usually within a range of from about 1 to about 1000 mg per day or, preferably, from about 10 to about 500 mg per day may be administered to an adult once or more times a day. In the case of administration in the form of injection, the active ingredient within a range of from about 0.1 to about 500 mg or, preferably, from about 3 to about 100 mg per day may be administered once or more times daily.

The above description concerns the utilization of the DNA participating in regulation of expression of PEBP2αA gene to a screening system for finding out an expression regulators for PEBP2αA gene. However, the DNA participating in the regulation of expression can also be utilized in the following purposes as well. Thus:
a) It can be used as a tool for finding out a factor binding to a promoter (low-molecular compound and proteinous factor), a factor which regulates the activity of promoter and a factor which regulates the binding of the above-mentioned factor to a promoter.
   An example of specific means for finding out a proteinic factor is a yeast (cell) 1-hybrid system (Clontech) which is a system by which an interaction in yeast or cell can be detected. By using a 1-hybrid system, in which a promoter region of PEBP2αA gene is used as a probe, one can find out a gene encoding for a factor (transcription enhancing factor) which enhances transcription by binding to a promoter region of a DNA, from a cDNA library which is derived from any cell or tissue and integrated into vectors prepared for a 1-hybrid method system. One can also use an expression cloning method (south western method using an expression library λ gt11 or the like) which has been used conventionally.
b) Since a promoter is a DNA, there is a possibility that a factor (including a compound or the like) binding to the promoter may be purified by an experiment utilizing affinity. Accordingly, the promoter can be used as a material of a DNA affinity column for purification of a binding factor.
c) When a transcription enhancing factor for PEBP2αA as mentioned in the above a) is obtained, there is high possibility that the promoter activity is regulated when it is a transcription factor or the like. If such a factor can be expressed as a protein, it is possible to screen a compound which can change the affinity between the factor and a promoter DNA, by an apparatus which analyzes the interaction between the substances (such as Biacore 2000). Accordingly, the promoter can be utilized for the screening of a compound which changes the affinity between the promoter and a transcription factor.
d) As mentioned previously, PEBP2αA (I) is supposed to be exclusively expressed in relatively differentiated osteoblast lineage and, therefore, there is a possibility that the promoter of PEBP2αA (I) gene is a promoter which specifically functions in relatively differentiated osteoblast lineage. When the promoter is applied to a gene therapy, a specific protein which is specific to the cells of osteoblast lineage can be expressed and can affect the surrounding tissues (such as hematopoietic cells in bone marrow, cancer which is metastasized to cartilage and bone). Examples of such specific proteins are cytokines, a growth factor for cell proliferation, a suppression factor for cell proliferation, a differentiation regulating factor, and a soluble receptor. When such a protein can be expressed in a bone-specific manner, promotion of hematopoiesis and promotion of proliferation or differentiation of chondrocytes can be expected.
e) When PEBP2αA promoter which functions specifically in osteoblast lineage is applied to the gene therapy like in the case of above d), a specific protein which is specific to the cells of the osteoblast lineage can be expressed to be used for differentiation or growth of the cell belonging to the osteoblast lineage itself. Examples of the specific proteins besides those mentioned in the above d) may be a receptor existing in cell membrane, a factor participating in signal transduction and a transcription factor. The subjects to be treated can be expanded to race horses, livestock and pets, as well as human being. The expected effects are stimulation of osteogenesis (improvement in strength of bone) and promotion of bone growth.
f) When the above-mentioned PEBP2αA promoter which is specific to the osteoblast lineage is utilized in the gene therapy by means of an autologous cell transplantation in the therapy of bone damage and bone fracture, it is expected to promote the repair. Examples of the proteins to be expressed are factors which promote the proliferation of cells which grow during the repair.
g) A knockin system can be used as another means for screening the expression regulator for PEBP2αA gene (Dev. Biol. 196(2), 218-227 (1998); Nat. Genet., 15(3), 303-306 (1997)) and the DNA of the present invention can be used in such a system as well.

### BRIEF EXPLANATION OF DRAWINGS

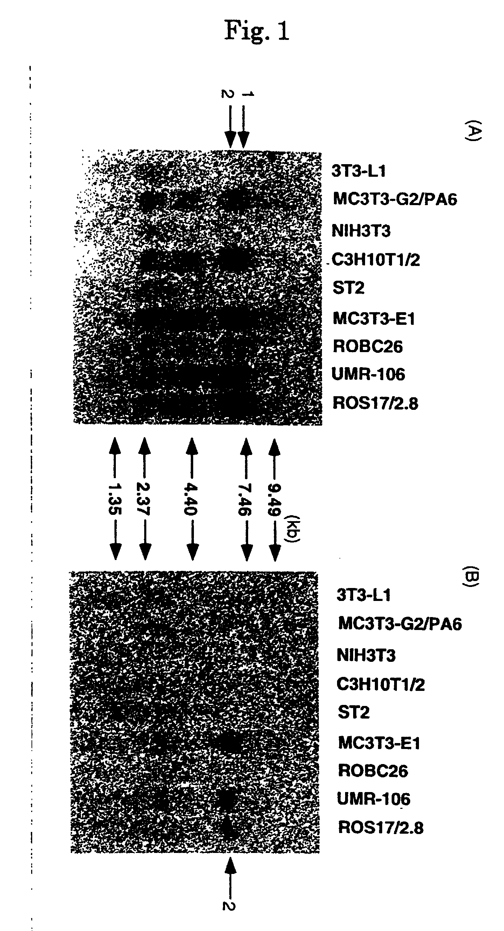

Fig. 1 shows photographs of electrophoresis showing an expression profile of PEBP2αA gene in various cell lines by northern hybridization. (A) shows the result when a region corresponding to nucleotide of 1293-1688 of Genbank Accession Number:D14636, the base sequence common to the genes of PEBP2αA (I) and (II) (a region coding for protein) is used as a probe while (B) shows the result when a probe which is specific to PEBP2αA (I) gene is used. In the drawing, 3T3-L1 and MC3T3-G2/PA6 are pre-adipocyte cell lines of mouse, NIH3T3 is a fibroblast cell line derived from fetus of mouse, C3H10T1/2 is an undifferentiated mesenchymal cell line derived from fetus of mouse, ST2 is a stroma cell line of bone marrow of mouse, MC3T3-E1 is a cell line derived from calvaria of mouse, ROBC26 is a pre-osteoblastic cell line of rat, UMR-106 is an osteosarcoma cell line of rat, and ROS17/2.8 is an osteosarcoma cell line of rat. In the drawing, the length of band 1 corresponds to about 7 kb while that of band 2 corresponds to about 6 kb.

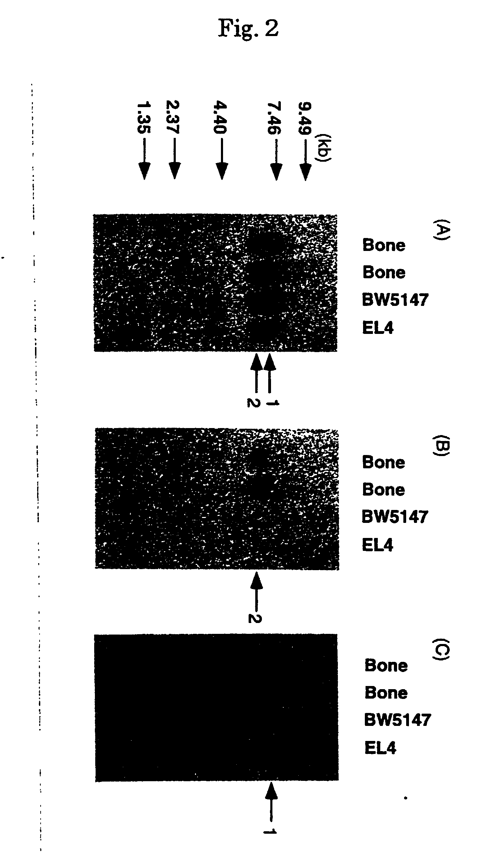

Fig. 2 shows the photographs of electrophoresis showing an expression profile of PEBP2αA gene in various cell lines and bone tissues by northern hybridization. (A) shows the result when a region corresponding to nucleotide of 1293-1688 of Genbank Accession Number:D14636, the base sequence common to the genes of PEBP2αA (I) and (II) (a region coding for protein) is used as a probe, (B) shows the result when a probe specific to PEBP2αA (I) gene used, and (C) shows the result when a probe which is specific to PEBP2αA (II) gene is used. In the drawing, Bone means bone tissue, BW5147 means a T lymphocyte cell line and EL4 means a B lymphocyte cell line. In the drawing, the length of band 1 corresponds to about 7 kb while that of band 2 corresponds to about 6 kb.
Fig. 3 is a schematic chart showing a signal transduction pathway of BMP.

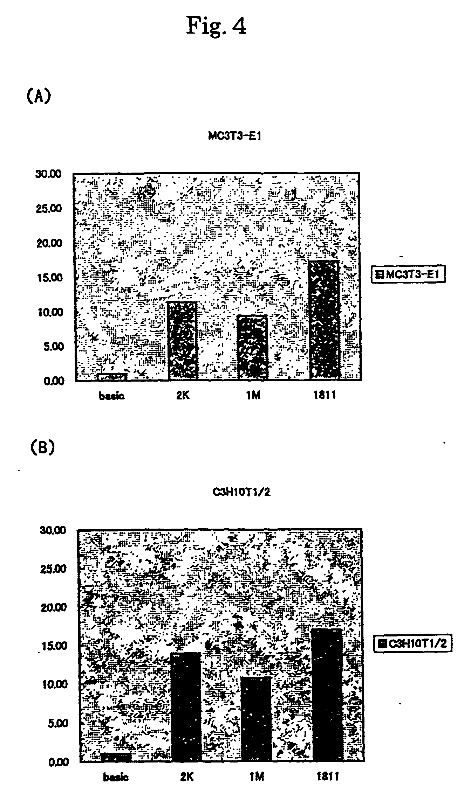

Fig. 4 is the graphs showing a promoter activity of PEBP2αA gene by a luciferase assay. (A) shows the result when MC3T3-E1 which is a cell line derived from calvaria of mouse is used, while (B) shows the result when C3H10T1/2 which is an undifferentiated mesenchymal cell line derived from fetus of mouse is used. In the drawing, 2K, 1M, 1811 and basic show the results of pGL3/2K, pGL3/1M, pGL3/1811 and pGL3basic used, respectively. Each value is given in a relative value of luciferase activity when the value upon introduction of pGL3basic is defined as 1.

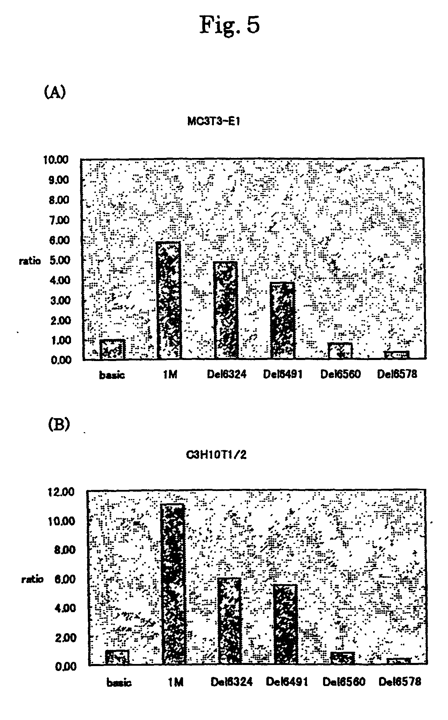

Fig. 5 is the graphs showing a promoter activity of PEBP2αA gene by a luciferase assay. (A) shows the result when MC3T3-E1 which is a cell line derived from calvaria of mouse is used, while (B) shows the result when C3H10T1/2 which is an undifferentiated mesenchymal cell line derived from fetus of mouse is used. In the drawing, 1M, De16324, De16491, De16560, De16578 and basic show the promoter activities when pGL3/1M, pGL3/De16324, pGL3/De16491, pGL3/De16560, pGL3/De16578 and pGL3basic are introduced, respectively. Each value is given in a relative value of luciferase activity when the value upon introduction of pGL3basic is defined as 1.

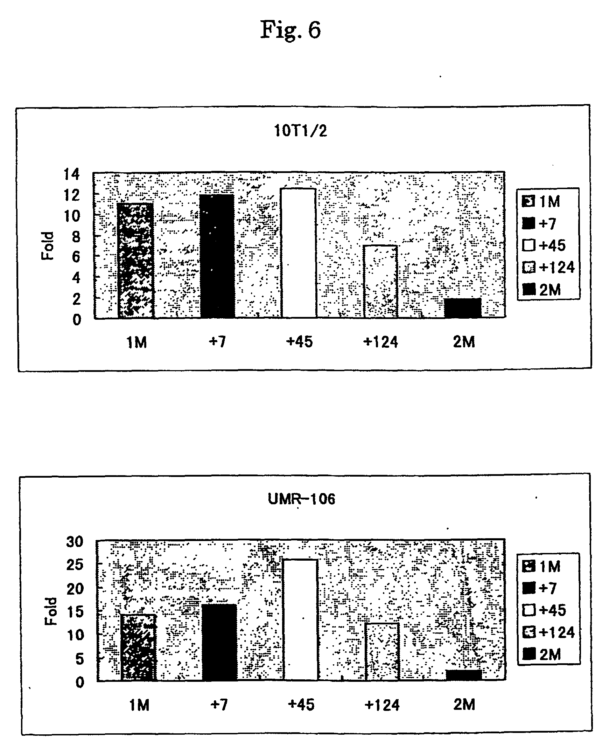

Fig. 6 is the graphs showing the relative luciferase activities of each of the vectors pGL3/1M, pGL3/+7, pGL3/+45, pGL3/+124 and pGL3/2M in C3H10T1/2 cell (10T1/2) and UMR-106 cell wherein the activity of pGL3basic is defined as 1.

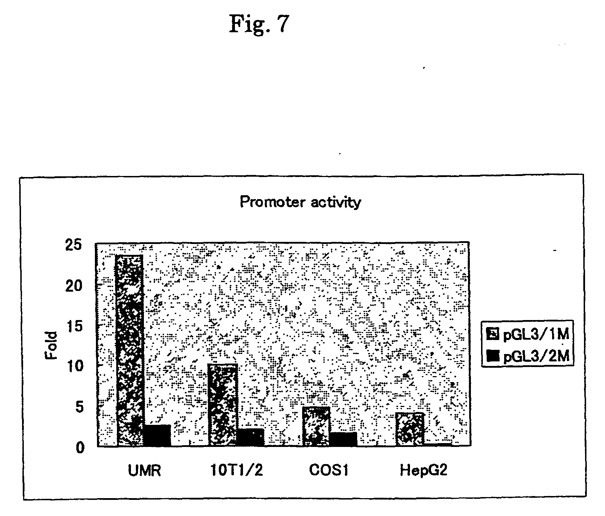

Fig. 7 is the graphs showing the relative luciferase activities of each of the vectors pGL3/1M and pGL3/2M in UMR-106 cell (UMR), C3H10T1/2 cell (10T1/2), COS1 cell and HepG2 cell when the activity of pGL3basic is defined as 1.

### BEST MODE FOR CARRYING OUT THE INVENTION

The present invention will now be further illustrated by way of the following examples although the present invention is not limited by those examples at all.

### Example 1.

### Cloning of Expression Regulatory region of PEBP2αA (I) Gene and Construction of Luciferase Vector

Based upon the amino acid sequence disclosed in the report by Karsenty, et al. (Cell, 89, 747-754 (1997)), a degenerate oligo-DNA (PEBone:5'-CCN CAY AAR CAR CCN CAR AAY CAY AAR TGY-3' where N is G, A, T or C; Y is C or T; and R is A or G) (SEQ ID NO:6) corresponding to the sequence from the 5th proline to the 14th cysteine was synthesized. PCR was carried out using this oligo-DNA and antisense DNA (AA:5'-TGG TGC GGT TGT CGT GCG GC-3') (SEQ ID NO:7) of the positions 1276-1295 of the previously-reported mouse PEBP2αA cDNA (Genbank No.D14636) as primers. Composition of the reaction mixture was 1 × XL buffer II, 0.2mM of each dNTPs, 1.2 mM of Mg(OAc)₂, 0.2µM of PEBone primer, 0.2µM of AA primer, 1µl of mouse bone cDNA (1µl of 100µl of the cDNA synthesized by a reverse transcription using a random primer with 0.1µg of mouse polyA(+) RNA isolated by a conventional method from bone tissue of mouse) and 2 units of rTth DNA polymerase XL (manufactured by Perkin-Elmer); volume of the reaction was 50µl; and a hot start method of PCR was employed using GeneAmp PCR System 9600 (manufactured by Perkin-Elmer) for 35 cycles where one cycle consisted of 94°C for 25 seconds and 64°C for 1 minute. Further, PCR was carried out with the same composition and reaction as mentioned above using 1µl of the above-prepared solution where antisense DNA of the positions 1036-1055 of the previously-reported cDNA (Genbank No. D14636) (S1:5'-TGA AGC GCC GGC TGG TGC TC-3') (SEQ ID NO:8) and the above-mentioned PEBone primer as primers. After confirmation of amplification of the DNA by means of polyacrylamide gel electrophoresis, the migrated PCR product was cut out, extracted/purified in a PAGE extraction buffer and directly subjected to sequencing by ABI Sequencer to determine the base sequence.

Based upon the sequence determined by the above-mentioned sequencing, isolation of a mouse PEBP2αA gene promoter region was attempted using Genome Walker Kit (Clontech) according to the protocol attached thereto using an antisense oligo-DNA (K5'3'-6;5'-TAA CCA TTT AAA CGC CAG AGC CTT CTT-3') (SEQ ID NO:9) corresponding to the sequence from the 15th glycine to the 23rd cysteine and an antisense oligo-DNA (K5'3'-7;5'-GCA GTC TTC CTG GAG AAA GTT TGC ACC-3') (SEQ ID NO:10) corresponding to the sequence from the 24th lysine to the 32nd leucine as primers. Briefly, PCR was carried out under the following condition using a PvuII library contained in the above kit as a template and using the primer AP1 contained in the kit and the above-mentioned K5'3'-6. Thus, composition of the reaction mixture was 1 × Tth PCR reaction solution, 0.2mM of each dNTPs, 1.1 mM of Mg(OAc)₂, 0.2µM of AP1, 0.2µM of K5'3'-6, 1µl of PvuII library and 1 × Advantage Tth Polymerase Mix; volume of the reaction was 50µl; and PCR was carried out using GeneAmp PCR System 9600 (manufactured by Perkin-Elmer) 7 cycles wherein one cycle consisted of 94°C for 2 seconds and 72°C for 3 minutes and 32 cycles wherein one cycle consisted of 94°C for 2 seconds and 67°C for 3 minutes. Additional PCR was carried out under the same reaction mixture composition as mentioned above using 1µl of this reaction solution as a template and using AP2 primer contained in the Kit and the already synthesized K5'3'-7 for 5 cycles wherein one cycle consisted of 94°C for 2 seconds and 72°C for 3 minutes and 20 cycles wherein one cycle consisted of 94°C for 2 seconds and 67°C for 3 minutes. The PCR product was subjected to an electrophoresis using 1% agarose gel, the amplified band of about 2 kbp was cut out and DNA fragment was purified using Prep A Gene Purification Kit (manufactured by Bio Rad). This DNA fragment was cloned using pGEM-T-Easy Vector System (manufactured by Promega) (named pG11). By analyzing the base sequence of the cloned PEBP2αA DNA, the sequence of the region 1853 bp upstream from the start site of translation was determined. The full length of the sequence is shown in SEQ ID NO:1. Incidentally, in the base sequence depicted in SEQ ID NO:1, the first 35 bp is a sequence derived from the adaptor sequence of the kit.

Subsequently, PCR was carried out again using a primer (KS5; 5'-ACT ATA GGG CAC GCG TGG TC-3') (SEQ ID NO:11) corresponding to the sequence of positions 1-20 of the base sequence depicted in SEQ ID NO:1 and an antisense primer (KA3; 5'-TTG CAA GCA CTA TTA CTG GAG-3') (SEQ ID NO:12) corresponding to the sequence of positions 1811-1791 thereof. Composition of the reaction mixture was 1× Pfu PCR reaction buffer, 0.2 mM of each of dNTPs, 0.2µM of KA3, 0.2µM of KS5, 10 ng of pG11, and 0.2U of Pfu DNA polymerase; volume of the reaction was 50µl; and a reaction was carried out using GeneAmp PCR System 9600 (manufactured by Perkin-Elmer) at 94°C for 40 seconds, and then for 15 cycles wherein each cycle consisted of 94°C for 30 seconds, 57°C for 42 seconds and 72°C for 2 minutes and, thereafter, at 72° for 7 minutes. The PCR product was subjected to an electrophoresis using 0.7% agarose gel, the amplified DNA fragment of 1811 bp was cut out and purified using Prep A Gene Purification Kit. This DNA fragment was inserted into the SmaI site of pGL3-Basic Vector (manufactured by Promega) to construct a luciferase vector pGL3/1811. The pGL3/1811 comprises the sequence of positions 1-1811 of the base sequence depicted in SEQ ID NO:1. Further, a fragment of 720 bp (positions 1113-1832 of SEQ ID NO:1) produced by digestion of the above-mentioned pG11 with a restriction enzyme Hind III was inserted into the Hind III site of pGL3-Basic Vector to construct pGL3/720H. With regard to the two luciferase vectors (pGL3/1811 and pGL3/720H) constructed as such, their directions were confirmed and their base sequences were determined, and then they were prepared in large quantities.

### Example 2.

### Cloning of Expression Regulatory region of PEBP2αA (II) Gene and Construction of Luciferase Vector

Antisense DNA (5'-RACE1; 5'-CCG GGC TCA CGT CGC-3'; 5'-terminal being phosphorylated) (SEQ ID NO:13) of the positions 1091-1106 of the sequence (Accession Number: D14636) registered in Genbank as mouse PEBP2αA cDNA was synthesized as a phosphorylated primer while four oligonucleotide DNAs [S1: positions 1055-1036 antisense 5'-TGA AGC GCC GGC TGG TGC TC-3' (SEQ ID NO:14), S2: positions 1045-1026 antisense 5'-GCT GGT GCT CGG ATC TAC AG-3' (SEQ ID NO:15), A1: positions 1061-1080 sense 5'-CCC TCC AGC AGC CTG CAG CC-3' (SEQ ID NO:16), and A2: positions 1071-1090 5'-GCC TGC AGC CCG GCA AGA TG-3' (SEQ ID NO:17)] were synthesized as PCR primers, and a cloning of 5'-terminal of the above-mentioned mouse PEBP2αA cDNA registered at the Genbank was attempted by the use of 5'-Full Race Core Kit (mannufactured by Takara Shuzo). Unexpectedly, there were many clones which contained only up to 25 bases upstream (position 991) from the start site of translation (position 1016), which suggested that a start site of transcription may exist also in the neighbourhood of this point.

Taking the above result into consideration, the inventors constructed two luciferase vectors. Thus, the positions 1-1015 and 319-1015 of the sequence (Accession Number: D14636) registered in Genbank were amplified by means of PCR using 0.1µg of mouse PEBP2α A genomic DNA (supplied from Dr. Toshifumi Komori, Faculty of Medicine, Osaka University; incidentally, a mouse genomic DNA (Clontech) may be used instead of that) as a template. The primers used were S1/28: 5'-CTG AAG TTA ACA ACG AAA AAT TAA CGC G-3' (SEQ ID NO:18), S319/340: 5'-GGC ACT TTG CAA AGA GCA GGA G-3' (SEQ ID NO:19) and AS992/1015: 5'-CAC AAC AGC CAC AAG TTA GCG AAG-3' (SEQ ID NO:20) (where S is a sense strand and AS is an antisense strand); composition of the reaction mixture was 1 × XL buffer II, 0.2 mM of each of dNTPs, 1.2 mM of Mg(OAc)₂, 0.2µM of each of the primers (combination of S1/28 with AS992/1015 and combination of S319/340 with AS992/1015 were used), 1µl of a mouse PEBP2αA genome DNA and 2 units of rTth DNA polymerase XL; volume of the reaction was 50µl; and a hot start PCR method was employed using GeneAmp PCR System 9600 (manufactuted by Perkin-Elmer) for 15 cycles wherein each cycle consisted of 94°C for 30 seconds and 65°C for 80 seconds. Subcloning of the amplified fragment into pGEMT-easy (manufactured by Promega) was carried out by TA cloning method and was cut out by digesting with EcoRI and inserted into the SmaI site of pGL3-Basic Vector (manufactured by Promega) after making the ends blunt using DNA blunting kit (manufactured by Takara Shuzo). The resulting two luciferase vectors were named pGL3/1015 and pGL3/697. The 1015 bp corresponding to the insert (a PCR-amplified fragment) of pGL3/1015 is shown in SEQ ID NO:2. pGL3/697 comprises 697 bp of the positions 319-1015 of the base sequence depicted in SEQ ID NO:2. After confirmation of those base sequences and directions, they were prepared in large quantities.

### Example 3.

### Luciferase Assay

A luciferase assay was carried out by means of incubation in a 12-well cell culture plate using a rat osteosarcoma cell line UMR-106 (obtained from Dainippon Pharmaceutical), a rat osteosarcoma cell line ROS17/2.8 (Institute of Physical and Chemical Research, Cell Bank), MC3T3-E1 derived from mouse calvaria (supplied from Dr. Kumegawa, Department of Dentistry, Meikai University), a primary culture calvaria cell of mouse (prepared according to Calif. Tissue Int., 38, 143-154 (1986)) and an undifferentiated mesenchymal cell line derived from fetus of mouse C3H10T1/2 (Institute of Physical and Chemical Research, Cell Bank). UMR-106 and ROS17/2.8 cells were cultured in a DMEM medium (manufactured by Cosmo Bio) containing 10% of fetal bovine serum (manufactured by Gibco), MC3T3-E1 cells and rat primary culture calvaria cells were cultured in an α-MEM medium (manufactured by Gibco BRL) containing 10% of fetal bovine serum (manufactured by Gibco) and C3H10T1/2 cells were cultured in a BME medium (manufactured by Gibco BRL) containing 10% of fetal bovine serum (manufactured by Gibco).

As a DNA mixture to be introduced, a mixture comprising 0.25µg of each of luciferase vectors (pGL3/72OH, pGL3/1811, pGL3/697 and pGL3/1015 prepared in Examples 1 and 2) and 0.33µg of a β-galactosidase expression vector (PCH110; manufactured by Pharmacia) per well were prepared. pBluscribeM13+ plasmid (manufactured by Stratagene) was added to the above DNA mixture to make the amount of the DNA for each dish become 1µg in total and, at the time when each cell reached a 40-60% confluent, transfection of above DNA mixture was carried out using LT-1 (manufactured by Panvera).

Gene introduction into each of the cells by LT-1 was carried out for 4 hours, the culture medium was exchanged, and incubation was continued for additional 44 hours. After completion of the incubation, the cells were lyzed in 100µl or 150µl of Reporter Lysis Buffer (manufactured by Promega) and 35µl of this solution was used for measuring the β-galactosidase activity by a conventional method to confirm the efficiency of DNA transfection in the cells. The luciferase activity was detected by measuring the strength of luminescence within 10 seconds using 15µl of the cell lysate and a luciferase assay reagent (manufactured by Promega) as a substrate in a luminometer ML3000 (manufactured by Dynatech Laboratories). The promoter activity of each vector was given by a relative value obtained by dividing a luciferase activity by a β-galactosidase activity and defining the value for a promoter-less pGL3-Basic vector as 1.

The result is shown in Table 2 in which Rat Primary stands for rat primary culture calvaria cells. In all of the vectors, promoter activity was detected and, in all of the cells tested, promoter activity was also detected. It was also found that the promoters of PEBP2αA (I) genes contained in pGL3/720H and pGL3/1811 have stronger activity than the promoters of PEBP2αA (II) genes contained in pGL3/697 and pGL3/1015. In addition, in all of the promoters, the longer ones (pGL3/1811 and pGL3/1015) showed stronger activity.

**Table 2**

| | pGL3-Basic | pGL3/720H | pGL3/1811 | pGL3/697 | pGL3/1015 |
|---|---|---|---|---|---|
| MC3T3-E1 | 1 | 13.4 | 80.6 | 7.0 | 8.2 |
| ROS17/2.8 | 1 | 10.6 | 17.4 | 3.5 | 3.4 |
| UMR-106 | 1 | 22.9 | 35.8 | 9.3 | 7.8 |
| Rat Primary | 1 | 12.2 | 32.9 | 5.2 | 7.5 |
| C3H10T1/2 | 1 | 8.5 | 18.9 | 3.1 | 5.0 |

### Example 4.

### Regulation of Promoter Activity by Fat-Soluble Ligand

Effect of each fat-soluble ligand on the promoter activity of the above-mentioned PEBP2αA (I) gene and PEBP2αA (II) gene was investigated.

First, as a receptor DNA for each fat-soluble ligand, 1) retinoic acid receptor expression vector; 2) vitamin D receptor expression vector; and 3) estrogen receptor expression vector were prepared. Since a retinoic acid receptor and a vitamin D receptor function after formation of a heterodimer with a retinoid X receptor, a retinoid X receptor expression vector was prepared as well. Each receptor expression vector was constructed as follows. Thus, an appropriate primer was synthesized based upon the reported estrogen receptor cDNA sequence (Nature, 320, 134-139 (1986)) in the case of an estrogen receptor expression vector; based upon the known vitamin D receptor cDNA sequence (Proc. Natl. Acad. Sci. USA, 85, 3294-3298 (1988)) in the case of a vitamin D receptor expression vector; based upon the known retinoic acid receptor cDNA sequence (Nature, 330, 624-629 (1987)) in the case of retinoic acid receptor expression vector; and based upon the known retinoid X receptor cDNA sequence (Cell, 68, 377-395 (1992)). Then the cDNA was cloned by means of PCR or the like and, after that, it was ligated to pSG5 (manufactured by Stratagene).

Primary cultured osteoblasts were incubated in an α MEM medium (manufactured by Gibco BRL) containing 10% of fetal bovine serum (manufactured by Gibco). Upon passage, the cells were plated in a 12-well culture plate using a phenol red-free α MEM medium (manufactured by Gibco BRL) containing 10% of fetal bovine serum (Gibco), from which steroidal hormones were removed by means of a treatment with a dextran-coated charcoal.

As a DNA mixture to be introduced, a mixture of 0.25µg of each luciferase vector (pGL3/720H, pGL3/1811, pGL3/697 and pGL3/1015 prepared in Examples 1 and 2), 0.05µg of each receptor expression vector and 0.33µg of a β-galactosidase expression vector (pCH110; manufactured by Pharmacia) per well was prepared. To such a DNA mixture was added pBluscribe M13+ plasmid (manufactured by Stratagene) so as to make the DNA amount for each well become 1µg in total and, at the time that the cells became 40-60% confluent, a gene transfection was carried out for 4 hours using LT-1 (manufactured by Panvera).

After the gene transfection, the medium was changed and all-trans-retinoic acid was added as a ligand to make final concentration 1µM to the wells into which retinoic acid receptor expression vector and retinoid X receptor expression vector were transfected; 1,25(OH)₂D₃ was added as a ligand to make 100nM to the wells into which vitamin D receptor expression vector and retinoid X receptor expression vector were transfected; and 17β-estradiol was added as a ligand to make 100nM to the wells into which estrogen receptor expression vector was introduced. At the same time, a control wells to which no ligand was added was prepared as well. After incubating for additional 44 hours, a luciferase activity was measured by the same method as in Example 3.

Each promoter activity is shown in Table 3. Similary to Example 3, the upper line shows the result shown by a relative value when the pGL3-Basic vector ("Basic" in the table) having no promoter activity was defined as 1, while the lower line shows the result shown by a relative value of the effect by addition of a ligand when the value in the absence of ligand was defined as 1 (in the line of "fold induction" in the table). In the table, RAR/RXR stands for co-introduction of a retinoic acid receptor expression vector and a retinoid X receptor expression vector; VDR/RXR stands for co-introduction of a vitamin D receptor expression vector and a retinoid X receptor expression vector; and ER α stands for introduction of an estrogen receptor expression vector. As a result, it was found that PEBP2αA (I) gene promoter activity increased by addition of retinoic acid; that PEBP2αA (I) gene and PEBP2αA (II) gene promoter activities were suppressed by addition of vitamin D; and that PEBP2αA (I) gene promoter activity was suppressed by addition of estrogen. As such, it was shown that the promoter activity of PEBP2αA gene was regulated by addition of the ligands.

**Table 3**

| | Bacic | pGL3/720H | | pGL3/1811 | | pGL3/697 | | pGL3/1015 | |
|---|---|---|---|---|---|---|---|---|---|
| Ligand (ligand: all-trans-retinoic acid; 1µM) | - | - | + | - | + | - | + | - | + |
| RAR/RXR | 1 | 18.7 | 34.1 | 53.5 | 87.6 | 4.7 | 5.3 | 8.0 | 7.5 |
| fold induction (ligand: 1,25(OH)₂D₃; 100 nM) | | | 1.8 | | 1.6 | | 1.1 | | 0.9 |
| VDR/RXR | 1 | 15.7 | 5.9 | 50.4 | 18.4 | 4.3 | 1.9 | 7.1 | 2.8 |
| fold induction (ligand: 17β-estradiol; 100 nM) | | | 0.38 | | 0.37 | | 0.46 | | 0.39 |
| ERα | 1 | 14.5 | 10.1 | 43.4 | 30.5 | 4.3 | 4.1 | 7.1 | 6.1 |
| fold induction | | | 0.70 | | 0.70 | | 0.95 | | 0.86 |

### Example 5.

### Regulation of Promoter Activity by BMP-4

Effect of BMP-4 which is one of osteogenetic factors on the promoter activity of PEBP2αA (I) gene and PEBP2αA (II) gene was investigated.

At first, primers were synthesized based upon the known BMP-4 cDNA sequence (Biochem. Biophys. Res. Commun. 186(3), 1487-1495 (1992)) and PCR was carried out using the primers to clone the cDNA of BMP-4. After that, the above BMP-4 cDNA was inserted into the pCDNA3.1(+) vector (manufactured by Invitrogen) whereby an expression vector of BMP-4 was constructed.

As to a cell for production of BMP-4, 293 cell derived from kidney of human fetus (Dainippon Pharmaceutical) was used. First, the cell was incubated in a culture dish having a diameter of 100 mm using a DMEM medium (manufactured by Gibco) containing 10% of fetal bovine serum (manufactured by Dainippon Pharmaceutical). Upon passage, an adjustment was done so as to make the cell numbers per one culture dish 4 × 10⁶ and incubation was carried out for 20 hours. After that, 5µg of the above expression vector were introduced therein using Lipofectamine (manufactured by Gibco). After 5 hours, the medium was changed and incubation was carried out for additional 42 hours . At the time of the completion of the incubation, the supernatant was recovered in a centrifugal tube and centrifuged at 12000 rpm for one minute and a supernatant thereof was recovered and used as a supernatant containing BMP-4.

In a luciferase assay, incubation was carried out on a 12-well cell culture plate using an undifferentiated mesenchymal cell line C3H10T1/2 derived from fetus of mouse (Institute of Physical and Chemical Research, Cell Bank). The incubation was carried out on a BME medium (manufactured by Gibco BRL) containing 10% of fetal bovine serum (manufactured by Gibco).

As a DNA mixture to be introduced, a DNA mixture comprising 0.25µg of pGL3/1811 or pGL3/1015 and 0.33µg of a β-galactosidase expression vector (PCH110; manufactured by Pharmacia) per well was prepared. To the DNA mixture was added pBluscribe M13+ plasmid (manufactured by Stratagene) to make the amount of DNA per dish become 1µg in total and, at the time when the cells for the assay became 40-60% confluent, a gene transfection was carried out using LT-1 (manufactured by Panvera).

After 4 hours from the gene transfection, the medium was changed to a medium comprising the same amounts of the above supernatant containing BMP-4 and a BME medium (manufactured by Gibco BRL) containing 10% of fetal bovine serum (manufactured by Gibco), and an incubation was carried out for five days. At the same time, a control group in which the medium was changed with a mixed medium containing the same amounts of the supernatant after culture of 293 cells producing no BMP-4 and a BME medium (manufactured by Gibco BRL) containing 10% of fetal bovine serum (manufactured by Gibco). At the time of 48 hours incubation, the medium was changed for a medium of the same composition. At the time of the completion of the incubation, the cells were lyzed in 150µl of Reporter Lysis Buffer (manufactured by Promega) and the β-galactosidase activity was measured by a conventional method using 35µl of this solution, whereby the efficiency of introduction of DNA into the cells was determined. A luciferase activity was detected by measuring the strength of luminescence during 10 seconds by a luminometer ML3000 (manufactured by Dynatech Laboratories) using 15µl of the above cell solution and a luciferase assay reagent (manufactured by Promega) as a substrate. Promoter activity of each vector was shown by a relative value obtained by dividing the luciferase activity by a β-galactosidase activity and difining the value of promoter-less pGL3-Basic vector as 1.

The result is shown in Table 4. The result shown by a relative value obtained by defining the value in the pGL3-Basic vector ("Basic" in the table) having no promoter activity as 1 is given in the upper line (in the line of "Relative Value" in the table) while the result shown by a relative value of the effect by addition of BMP-4, which is obtained by defining the value in the absence of BMP-4 as 1, is given in the lower line (in the line of "fold induction" in the table). As a result, it was found that both promoters for PEBP2αA (I) gene and PEBP2αA (II) gene have promoter activity and that the activity of the promoter was enhanced by the addition of BMP-4.

**Table 4**

| | Basic | pGL3/1811 | | pGL3/1015 | |
|---|---|---|---|---|---|
| BMP-4 | - | - | + | - | + |
| Relative Value | 1 | 209.4 | 354.4 | 9.6 | 19.6 |
| fold induction | | | 1.69 | | 2.04 |

### Example 6.

### Cloning and Sequencing of Genomeic Clone of PEBP2αA (I)

Genomic clone containing an expression regulatory region of PEBP2αA (I) gene cloned by PCR method in Example 1 was obtained and the base sequence of the genomic DNA was determined. The details are as follows.

### 1) Screening of mouse genome library.

E. coli XL-1 Blue MRA strain (obtained from Stratagene) was incubated overnight at 37°C in 50 ml of LB medium containing 0.2% of maltose and the cells were collected by means of centrifugation at 3000 rpm for 15 minutes and suspended in a 10mM MgSO₄ solution so as to make O.D.600 become about 0.5. To 500µl of this suspension were added 20µl of a phage library of mouse genome (obtained from Clontech; Cat#ML 1044j, Lot # 4511; titer was 1.5 × 10⁹ pfu/ml) diluted with SM buffer to become 500 pfu/µl, and an infection was performed at 37°C for 30 minutes. To this solution were added 7 ml of NZYM containing 0.7% of agarose kept at 60°C and the mixture was plated in a 15 cm plate onto which 40 ml of NZYM containing 1.5% of agarose were solidified. Incubation was carried out at 37°C for 7 hours and, when the plaque has grown to about 1 mm diameter, the plate was kept at 4°C.

Then a nylon membrane (Colony/Plaque Screen manufactured by NEN Life Science Products) was placed for 2 minutes on this plate so that the phage was adsorbed on the membrane. The membrane was floated on a solution of 0.5N NaOH and 1.5M NaCl for 2 minutes for alkaline denaturation and neutralized for 20 minutes in a 0.5M Tris-Cl (pH 7.4) and 1.5M NaCl solution. This was dried on a filter paper at 60°C so that the phage DNA was immobilized on the membrane.

A DNA containing the positions 1-1811 of the base sequence depicted in SEQ ID NO:1 cloned into pGEM-T Easy was digested in a restriction enzyme EcoRI, an electrophoresis in agarose gel was carried out and a DNA fragment of about 2 kbp was cut out and purified using Prep A Gene DNA Purification Kit (manufactured by Bio-Rad), whereupon a DNA probe was prepared. About 100 ng of this DNA probe were subjected to the method contained in BcaBEST Labeling Kit (manufactured by Takara Shuzo) to prepare a probe labeled with ³²P. The above-prepared membrane onto which a phage DNA was immobilized was hybridized with this probe according to the protocol attached to the membrane usage insruction, an excessive probe was washed out and an autoradiography was carried out. Such a screening was repeated for three cycles to give plural positive clones.

Each 5 × 10⁴ of the plural phages obtained in the above 1) were plated in a 15 cm plate by the same manner as above and incubated under high humidity until the plaques contacted each other. Then 8 ml of SM buffer were added thereto and the phage was eluted at room temperature for about one hour. The buffer (about 7 ml) with which the phage was eluted was recovered to a centrifugal tube and centrifuged at 5000 rpm for 10 minutes, 10U of DNase I were added to the supernatant and digestion was performed at 37°C for 30 minutes. To this were added 5 ml of a PEG solution (30% PEG, 2.5M NaCl) and the mixture was allowed to stand on ice for one hour and centrifuged at 13000 rpm for 15 minutes. The precipitate was dissolved in 500µl of SM buffer and extracted with phenol and with chloroform by a conventional manner, 1 ml of ethanol and 40µl of 3M sodium acetate were added thereto and the mixture was centrifuged at 13000 rpm for 10 minutes. The precipitate was dissolved in 300µl of a TE buffer, RNase was added thereto to make its concentration 20µg/ml and digestion was performed at 37°C for 30 minutes. This was again extracted with phenol and with chloroform, precipitation with ethanol was perfoemed, the precipitate was dissolved in 100µl of a TE buffer, and this was used as a phage DNA solution.

### 3) Southern analysis

In order to determine whether the plural phage clones obtained in the above 2) actually contained the expression regulatory region for PEBP2αA (I) gene, the following southern analysis was carried out.

First, about 1µg of the phage DNA obtained in the above screening was digested with restriction enzymes (BamHI, EcoRI, HindIII and SalI), subjected to an electrophoresis using 1% agarose gel, transferred by a capillary action to a Nylon membrane Hybond-N+ (manufactured by Amersham), and immobilized with UV of 1200µJ. On the other hand, a probe labeled with alkaline phosphatase (AP) was prepared using Gene Image (manufactured by Amersham) according to the protocol attached thereto using the DNA for probe which was used for the screening. This probe was hybridized with the above membrane by a conventional method, an excessive probe was washed out, a blocking was carried out to react with an. anti-AP antibody, an excessive antibody was washed out, and the membrane was made luminous by a luminous reagent and then autoradiographed.

As a result, it was found that the plural genomic clones obtained could be classified into two groups, and each of them contained an expression regulatory region (positions 1-1811 of SEQ ID NO:1) of PEBP2αA (I) gene.

### 4) Subcloning

About 10µg of the phage DNA obtained in 3) was digested with restriction enzymes (BamHI, SacI). This was subjected to an electrophoresis using 1% agarose gel, each DNA fragment was cut out and purified using Prep-A-Gene DNA Purification System (manufactured by Bio-Rad) according to the protocol attached thereto. The fragment was cloned into pBluescript KS. Among them, a clone of about 7kbp obtained by digestion with SacI and a clone of about 8kbp obtained by digestion with BamHI were named pP6S3-1 and pP1B2-11, respectively, and used in the following analysis.

### 5) Determination of base sequence

Base sequence was determined using the above two plasmids as templates by means of an automatic sequencer (ABI 377) according to the instruction attached thereto using the primers as shown in the following Tables 5-7. In the tables, "Name" stands for the name of the primer; "5'-term. position" stands for the position of the 5'-terminal of the primer; "S/AS" stands for a sense strand (normal strand) or an antisense strand (reverse strand); "Sequence" stands for particular base sequence of the primer; and "Seq.No." stands for SEQ ID NO of the sequence listing.

**Table 5**

| Name | 5'-term. position | S/AS | Sequence | Seq. No. |
|---|---|---|---|---|
| PKGS5 | 385 | S | 5'-CAAGTGAAAAGTCTTGTGAAT-3' | 21 |
| PKGS8 | 980 | S | 5'-GCTTAATAGAGACCTCAATCC-3' | 22 |
| PKGS10 | 1587 | S | 5'-TAAATTCATCAAAATTGCTGAAG-3' | 23 |
| PKGS19 | 2101 | S | 5'-CACAAAGACAAAATGACAATCAAGG-3' | 24 |
| PKGS20 | 2604 | S | 5'-ACATTCCTGGACAGAGCTGTC-3' | 25 |
| PKGS21 | 3211 | S | 5'-AGTTTGATAAATGCTAGGTG-3' | 26 |
| PKGS22 | 3714 | S | 5'-GCTGTATTTCATTGTTATTGC-3' | 27 |
| PKGS23 | 4207 | S | 5'-CAGACACAGGAAATAGGGTTAGG-3' | 28 |
| PKGS24 | 4698 | S | 5'-CAAACAAAAGAATGTATTTCTGTGG-3' | 29 |
| PKGS4 | 4814 | AS | 5'-CTGGAAAGACATGGTAATGC-3' | 30 |
| PKGS7 | 4207 | AS | 5'-GTGAGTTATGGTCTGTATCTC-3' | 31 |
| PKGS9 | 3602 | AS | 5'-TGAATATAACAGTATAGTAATATC-3' | 32 |
| PKGS11 | 3074 | AS | 5'-TTTTGAACTTGGCTATGAGGT-3' | 33 |
| PKGS12 | 2365 | AS | 5'-TGTGGGAAACATGCTTGTATG-3' | 34 |
| PKGS15 | 1866 | AS | 5'-CATTTGTTGTTGGGTAGTGGC-3' | 35 |
| PKGS16 | 1369 | AS | 5'-GGTGTTGCTGTCTATATCTCC-3' | 36 |
| PKGS17 | 921 | AS | 5'-AATGAAGCAGTGGAATACGG-3' | 37 |
| PKGS18 | 512 | AS | 5'-ACTGACATTTTTAGTGAACTC-3' | 38 |

**Table 6**

| Name | 5'-term. position | S/AS | Sequence | Seq. No. |
|---|---|---|---|---|
| PKGB11 | 8157 | S | 5'-GCTACCCAACATGTCCCAGC-3' | 39 |
| PKGB12 | 8641 | S | 5'-AGTAGTCACTGAGAGACAAGAACATT-3' | 40 |
| PKGB13 | 9179 | S | 5'-AGTACATGTGCCAATTTTGCA-3' | 41 |
| PKGB15 | 9807 | S | 5'-GATTTCACTTTTAAATCATAAAGATG-3' | 42 |
| PKGB22 | 9994 | S | 5'-AAGCTAATAATCACTGATGGTC-3' | 43 |
| PKGB17 | 11953 | S | 5'-GAATCTAATATACATCCAAGG-3' | 44 |
| PKGB19 | 13430 | AS | 5'-GAAATAATTCTAAATTATGTGGTAG-3' | 45 |
| PKGB16 | 11589 | AS | 5'-TGTTTCTTACATCCATTCAACAG-3' | 46 |
| PKGB23 | 11046 | AS | 5'-AGTTATACAACTTTGTTTCATTGG-3' | 47 |
| PKGB14 | 9149 | AS | 5'-GCACTGTAAGAAGGTAACCTTGAAGGGAGG-3' | 48 |
| PKGB7 | 7716 | AS | 5'-TGAATGTCTGATTAGTACAG-3' | 49 |

**Table 7**

| Name | 5'-term. position | S/AS | Sequence | Seq. No. |
|---|---|---|---|---|
| PKGB24 | 7541 | S | 5'-GAATTATTGAAACATTGTGG-3' | 50 |
| PKGB25 | 8798 | S | 5'-TAGGGGTGGGTCTTAAAGTTC-3' | 51 |
| PKGB26 | 9337 | S | 5'-GTGTCCTTGATCTCTGCTAC-3' | 52 |
| PKGB27 | 10638 | S | 5'-ATGAGTCTTGGGACGTGTGC-3' | 53 |
| PKGB28 | 11290 | S | 5'-TGATTAATGTGTTACAATCC-3' | 54 |
| PKGB29 | 12795 | S | 5'-CCTTTTTGGGTATCAGTCTG-3' | 55 |
| PKGB30 | 13285 | S | 5'-GCTGTTTTTTTAATAATAACTGC-3' | 56 |
| PKGB31 | 13004 | AS | 5'-TTTTGATTTTACAGATTGAC-3' | 57 |
| PKGB32 | 12568 | AS | 5'-GTGAGATAGCTGACCATAAG-3' | 58 |
| PKGB33 | 10519 | AS | 5'-AAGAAGCCTGTCCCATTGGAG-3' | 59 |
| PKGB34 | 9961 | AS | 5'-TACTAAAAGAACTAAAATATACT-3' | 60 |
| PKGB35 | 9431 | AS | 5'-AATCTTCTGTTTCAAAAGGATTG-3' | 61 |
| PKGB36 | 8877 | AS | 5'-TCTGAAGTTTGAGCAGATCTTGG-3' | 62 |
| PKGB37 | 8251 | AS | 5'-TCACTAGTTTGAAAATTCTG-3' | 63 |
| PKGB38 | 7614 | AS | 5'-CAGCTGAGAATGCTAACTCG-3' | 64 |

The determined base sequence comprising 13907bp is shown in SEQ ID NO:4. The positions 4834-6609 of the base sequence depicted in SEQ ID NO:4 corresponds to the positions 36-1811 of the base sequence depicted in SEQ ID NO:1, while the positions 4834-6651 of the base sequence depicted in SEQ ID NO:4 corresponds to the positions 36-1853 of the base sequence depicted in SEQ ID NO:1.

The initiation codon for translation (the first methionine) of PEBP2αA (I) (called Osf2/Cbfal in the following reference) described in Cell, 89, 747-754 (1997) corresponds to the positions 6652-6654 of the base sequence depicted in SEQ ID NO:4. In the meanwhile, there are reports in recent years that the initiation codon for translation of PEBP2αA (I) is not the first methionine but the second methionine (Proc. Natl. Acad. Sci. USA, 94, 8646-8651 (1997); Jikken Igaku, 16(2), 107-113 (1998)), and the second methionine corresponds to the positions 7053-7055 of the base sequence depicted in SEQ ID NO:4.

As a result of the sequence analysis, it was found that, in the region of the positions 4834-7052 of the base sequence depicted in SEQ ID NO:4, there are many binding sequences for PEBP2 family including PEBP2αA *per se*, for Sry belonging to an HMG family including SOX9 which is an important factor for differentiation of chondrocytes and for maintaining the characteristics of chondrocytes, and similar sequences thereto, and for GATA family which is believed to play a partial role in signal transduction of BMP, and whose expression is supposed to be regulated by BMP, and similar sequences thereto. Particular sequences are shown in Table 1.

### Example 7.

### Construction of Luciferase Vector Using Genomic DNA

The following two luciferase vectors were constructed using the genomic DNA depicted in SEQ ID NO:4.

### 1) Construction of pGL3/2K

The sequence of the positions 4834-6609 of SEQ ID NO:4 was amplified by PCR using a sense primer 2K3'B of the positions 4834-4855 of SEQ ID NO:4 having a BamHI recognition sequence at 5'-terminal (5'-TAC-GGA-TCC-TAA-AGA-ATC-TTA-TGA-ACA-TGA-T-3' [SEQ ID NO:65]) and an antisense primer 2K5'B of the positions 6609-6588 of SEQ ID NO:4 having a BamHI recognition sequence at 5'-terminal (5'-TAC-GGA-TCC-TTG-CAA-GCA-CTA-TTA-CTG-GAG-A-3'[SEQ ID NO:66]). Composition for the reaction mixture was 1 × PCR Buffer, 0.2mM of each of dNTPs, 0.2µM of each primer, 10 ng of pP6S3-1 (prepared in Example 5) and 2 units of Amplitaq DNA polymerase (manufactured by ABI); volume of the reaction was 50µl; and condition for the reaction was that GeneAmp PCR System 9600 (Perkin Elmer) was used and a cycle consisting of 94°C for 30 seconds, 60°C for 30 seconds and 72°C for 30 seconds was repeated 15 times. The resulting product was digested with a restriction enzyme BamHI and inserted into Bg1II site of pGL3-Basic vector. The luciferase vector prepared as such was named pGL3/2K. After that, direction and total base sequence of the inserted fragment were confirmed by determination of the base sequence of the inserted region containing the cloning site and then a large-scale production was carried out.

### 2) Construction of pGL3/1M

The sequence of the positions 4834-6651 of SEQ ID NO:4 was amplified by means of PCR in the same manner as mentioned above using a sense primer 2K3'B of the positions 4834-4855 of SEQ ID NO:4 having a BamHI-recognition sequence at 5'-terminal (5'-TAC-GGA-TCC-TAA-AGA-ATC-TTA-TGA-ACA-TGA-T-3' [SEQ ID NO:65]) and an antisense primer 1M5'B of the positions 6651-6626 of SEQ ID NO:4 having a BamHI-recognition sequence at 5'-terminal (5'-TAC-GGA-TCC-TCA-CAC-AAT-CCA-AAA-AAG-CAA-AAG-CT-3' [SEQ ID NO: 67]), digested with a restriction enzyme BamHI and inserted into Bg1II site of pGL3-Basic vector. The luciferase vector constructed as such was named pGL3/1M, confirmed in the same manner as mentioned and prepared in large quantities.

### Example 8.

### Luciferase assay

The following luciferase assays were carried out using the three luciferase vectors, i.e. pGL3/1811 prepared in Example 1 and pGL3/2K and pGL3/1M prepared in Example 7.

At first, MC3T3-E1 derived from mouse calvaria (supplied from Dr. Kumegawa, Department of Dentistry, Meikai University) and an undifferentiated mesenchymal cell line C3H10T1/2 derived from fetus of mouse (Institute of Physical and Chemical Research, Cell Bank) were plated on a 12-well cell culture plate so that the cell numbers become 2 × 10⁵ cells/12 wells. The plates were incubated in an α -MEM medium containing 10% of fetal bovine serum (manufactured by Gibco) and a BME medium (manufactured by Gibco BRL) respectively.

As a DNA mixture for introduction, a mixture of 0.5µg of each of the above mentioned luciferase vectors and 0.3µg of a β-galactosidase expression vector (PCH 110 manufactured by Pharmacia) per well was prepared. To the DNA mixture was added pBluscribe M13+ plasmid (manufactured by Stratagene) to make the DNA amount per well become 1µg in total and, at the time of 24 hours from plating of each of the cells, gene transfection was carried out using LT-1 (Panvera).

After 4 hours from the gene transfection by LT-1, the culture medium was changed for the above-mentioned one and incubation was carried out for two days. At the time of the completion of incubation, the cells were lyzed in 150µl of Reporter Lysis Buffer (manufactured by Promega) and, using 35µl of this lysate, β-galactosidase activity was measured by a conventional method to determine the efficiency of DNA transfection into the cell. Luciferase activity was detected by measuring the strength of luminescence within 10 seconds by a luminometer ML 3000 (Dynatech Laboratories) using 15µl of the cell lysate and a luciferase assay reagent (manufactured by Promega) as a substrate. Promoter activity of each vector was given by a relative value obtained by dividing the luciferase activity by β-galactosidase activity and defining the value for promoter-less pGL3-Basic as 1.

The result is shown in Fig. 4. In all of the vectors, promoter activity was detected, and the promoter activity was found in all of the cells tested.

### Example 9.

### Cloning and Seqencing of Genomic Clone of human PEBP2αA (I)

Human genomic clone corresponding to the sequence of mouse PEBP2αA (I) genome depicted in SEQ ID NO:4 was obtained and its base sequence was determined. To be more specific, a human promoter finder kit (manufactured by Clontech) was used and an experiment was carried out according to the PCR cycle in the protocol of the kit using AP1 primer (5'-GTA ATA CGA CTC ACT ATA GGG C-3') and AP2 primer (5'-ACT ATA GGG CAC GCG TGG T-3') contained in the kit and hCBFA1GSP1 (5'-AAG TTT TGC TGA CAT GGT GTC ACT GTG-3')(SEQ ID NO:68) and hCBFA1GSP2 (5'-CTG AAG AGG CTG TTT GAT GCC ATA GTC-3') (SEQ ID NO:69) as primers. Thus, composition for the reaction mixture was 1 × Tth PCR reaction buffer, 0.2 mM of each of dNTPs, 1.1 mM of Mg(OAc)₂, 0.2µM of AP1, 0.2µM of hCBFA1GSP1, 1µl of EcoRV library and 1× Advantage Tth Polymerase Mix; volume of the reaction was 50µl; and PCR was carried out with GeneAmp PCR System 9600 (Perkin-Elmer) 7 cycles where one cycle consisted of 94°C for 2 seconds and 72°C for 3 minutes, and 32 cycles where one cycle consisted of 94°C for 2 seconds and 67°C for 3 minutes. Further, 2µl of a 50-fold diluted solution of this reaction mixture were used as a template and PCR was carried out using a primer AP2 contained in the kit and the above-mentioned hCBFA1GSP2 (SEQ ID NO:69) at the same reaction mixture composition as above. PCR was performed 5 cycles where each cycle consisted of 94°C for 2 seconds and 72°C for 3 minutes and 25 cycles where each cycle consisted of 94°C for 2 seconds and 67°C for 3 minutes. This PCR product was subjected to an electrophoresis using 1% agarose gel, the amplified band of about 1.5 kbp was cut out, and the DNA fragment was purified using a QIAquick Gel Extraction Kit (manufactured by Qiagen). This was cloned using pGEM-T-Easy Vector System (manufactured by Promega). A base sequence was determined by an automatic sequencer (ABI 377) according to the protocol attached thereto using the resulting DNA as a template and various primers comprising part of the DNA depicted in SEQ ID NO:4.

The determined base sequence is shown in SEQ ID NO:5. The position 1 of the base sequence depicted in SEQ ID NO:5 corresponds to the position 5555 of the base sequence depicted in SEQ ID NO:4, while the position 1487 of the base sequence depicted in SEQ ID NO:5 corresponds to the position 7234 of the base sequence depicted in SEQ ID NO:4.

### Example 10.

### Determinations of Start Site of Transcription of PEBP2αA Genes

The start sites of transcription of PEBP2αA (I) and (II) genes were determined as follows.

### 1) Determination of start site of transcription of PEBP2αA (I) gene.

The start site of transcription was determined using Cap Site cDNA (Gene 138, 171-174 (1994)) where the start site of transcription can be easily determined by means of PCR, by uniquely substituting the cap structure existing at the 5'-terminal of mRNA with an oligo DNA. Cap Site cDNA derived from mouse bone tissue (manufactured by Nippon Gene) was used as a template and an amplification was carried out by Nested PCR. The first PCR was carried out using 1RC primer contained in the kit (5'-CAA-GGT-ACG-CCA-CAG-CGT-ATG-3') and an antisense primer K5'3'-1 of the positions 7074-7100 of SEQ ID NO:4 (5'-GCT-TTG-CTG-ACA-CGG-TGT-CACTGC-GCT-3')(SEQ ID NO:72). Composition of the reaction mixture was 1 × PCR buffer, 0.2 mM of each of dNTPs, 0.5µM of each primer, 1µl of Cap Site cDNA derived from bone tissue of mouse and 2 units of AmpliTaq DNA Polymerase (manufactured by Perkin Elmer); volume for the reaction was 50µl; and condition for the reaction was that GeneAmp PCR System 9600 (Perkin Elmer) was used and a cycle consisting of 94°C for 30 seconds, 57°C for 30 seconds and 72°C for 30 seconds was conducted 35 cycles. The second PCR was carried out using the resulting PCR product as a template. The second PCR was carried out using 2RC primer contained in the kit (5'-GTA-CGC-CAC-AGC-GTA-TGA-TGC-3') and an antisense primer K5'3'-3 consisting of the positions 7055-7084 of SEQ ID NO:4 (5'-GTC-ACT-GCG-CTG-AAG-AGG-CTG-TTT-GAC-GCC-3') (SEQ ID NO:73). Composition and volume of the reaction were the same as those for the first PCR and 0.5µl of the first PCR product was used as a template. Condition for the reaction was that GeneAmp PCR System 9600 (Perkin Elmer) was used and a cycle consisting of 94°C for 30 seconds, 57°C for 30 seconds and 72°C for 30 seconds was conducted 25 cycles.

Sequence of the resulting PCR product was determined using ABI Prism Big Dye Sequencing Kit and ABI Prism DNA Sequencer 377A (Applied Biosystem). As a result, it was found that the transcription started at A of the position 6659 of SEQ ID NO:4.

Further, the first PCR was carried out under the same condition as above using Cap Site cDNA derived from mouse bone tissue as a template and using 1RC primer contained in the kit (5'-CAA-GGT-ACG-CCA-CAG-CGT-ATG-3') and an antisense primer GSP3 consisting of the positions 6679-6703 of SEQ ID NO:4 (5'-AGTTTGCACCGCACTTGTGGTTCTG-3') (SEQ ID NO:74). Then, the second PCR was carried out in the same manner using 0.5µl of the first PCR product as a template and using 2RC primer contained in the kit (5'-GTA-CGC-CAC-AGC-GTA-TGA-TGC-3') and an antisense primer NGSP3 consisting of the positions 6646-6669 of SEQ ID NO:4 (5'-GTGAGGCGAATGAAGCATTCACAC-3') (SEQ ID NO:75). The resulting PCR product was subcloned into pGEM-T Easy Vector (manufactured by Promega), a plasmid was prepared using QIAquick Miniprep Spin Kit (manufactured by Qiagen) and the base sequence was determined using ABI prism Big Dye Sequencing Kit and ABI Prism DNA Sequencer 377A (Applied Biosystem). As a result, it was found that the transcription started at T of the position 6635 and also at A of the position 6596 of SEQ ID NO:4.

### 2) Determination of start site for transcription of PEBP2αA (II)

Cap Site cDNA derived from mouse mouse bone tissue (manufactured by Nippon Gene) was used as a template and an amplification was carried out by Nested PCR. The first PCR was carried out using the 1RC primer contained in the kit (5'-CAA-GGT-ACG-CCA-CAG-CGT-ATG-3') and an antisense primer Ito AS+1036 consisting of the positions 1036-1055 of the sequence registered in Genbank as a mouse PEBP2αA cDNA (Accession Number: D14636) (5'-TGA-AGC-GCC-GGC-TGG-TGC-TC-3') (SEQ ID NO:76). Composition for the reaction mixture was 1×PCR buffer, 0.2mM of each of dNTPs, 0.5µM of each primer, 1µl of Cap Site cDNA derived from mouse bone tissues and 2 units of AmliTaq DNA Polymerase (manufactured by Perkin Elmer); volume of the reaction was 50µl; and condition of the reaction was that GeneAmp PCR System 9600 (Perkin Elmer) was used and a cycle consisting of 94°C for 30 seconds, 57°C for 30 seconds and 72°C for 30 seconds was conducted 35 cycles. Then the second PCR was carried out using the resulting PCR product as a template. The second PCR was carried out using 2RC primer contained in the kit (5'-GTA-CGC-CAC-AGC-GTA-TGA-TGC-3') and an antisense primer Ito AS+1015 consisting of the positions 1015-1035 of the above sequence (5'-GGA-TCT-ACA-GGA-ATA-CGC-ATC-3') (SEQ ID NO:77). Composition and volume of the reaction mixture were the same as those for the first PCR and, as a template, 0.5µl of the first PCR product was used. Condition of the reaction was that GeneAmp PCR System 9600 (Perkin Elmer) was used and a cycle consisting of 94°C for 30 seconds, 57°C for 30 seconds and 72°C 30 seconds was conducted 25 cycles.

The resulting PCR product was subcloned into pGEM-T Easy Vector (manufactured by Promega), a plasmid was prepared using QIAquick Miniprep Spin Kit (manufactured by Qiagen), and the base sequence was determined using ABI Prism Big Dye Sequencing Kit and ABI Prism DNA Sequencer 377A (Applied Biosystem). As a result, it was found that, as expected in Example 2, a start site of transcription was present 25-base upstream (position 991 of SEQ ID NO:2) from the start site (position 1016) of translation.

### Example 11.

### Construction of pGL3/De16324, pGL3/De16491, pGL3/De16560 and pGL3/De16578 and Luciferase Assay

### 1) Construction of pGL3/De16324, pGL3/De16491, pGL3/De16560 and pGL3/De16578

In order to analyze the promoter activity in more detail, the following four vectors were constructed, which contain the sequence just before the first methionine existing at the position 6652 of the base sequence depicted in SEQ ID NO:4.

The sequences of the positions 6324-6651, the positions 6491-6651, the positions 6560-6651 and the positions 6578-6651 of the base sequence depicted in SEQ ID NO:4 were amplified by means of PCR using a sense primer De16324 consisting of the positions 6324-6346 of the base sequence depicted in SEQ ID NO:4 having a KpnI-recognition sequence at 5'-terminal (5'-ATG-GTA-CCA-GAG-GGA-GAA-AGG-GAG-AGA-GAG-3')(SEQ ID NO:78), a sense primer De16491 consisting of the positions 6491-6512 of the base sequence depicted in SEQ ID NO:4 having a KpnI-recognition sequence at 5'-terminal (5'-ATG-GTA-CCA-GAA-AGA-GGG-AGG-GAA-GAG-AGC-3')(SEQ ID NO:79), a sense primer De16560 consisting of the positions 6560-6581 of the base sequence depicted in SEQ ID NO:4 having a KpnI-recognition sequence at 5'-terminal (5'-ATG-GTA-CCG-TGA-GGT-CAC-AAA-CCA-CAT-GAT-3')(SEQ ID NO:80) or a sense primer De16578 consisting of the positions 6578-6599 of the base sequence depicted in SEQ ID NO:4 having a KpnI-recognition sequence at 5'-terminal (5'-ATG-GTA-CCT-GAT-TCT-GTC-TCT-CCA-GTA-ATA-3')(SEQ ID NO:81) and an antisense primer 1M5'B consisting of the positions 6651-6626 of the base sequence depicted in SEQ ID NO:4 having a BamHI-recognition sequence at 5'-terminal (5'-TAC-GGA-TCC-TCA-CAC-AAT-CCA-AAA-AAG-CAA-AAG-CT-3) (SEQ ID NO:67). Composition of the reaction mixture was 1 × KOD Buffer (#1), 0.2 mM of each of dNTPs, 0.2µM each of sense and antisense primers, 10 ng of pP6S3-1 (prepared in Example 6) and 2.5 units of KOD polymerase (manufactured by Toyobo); volume of the reaction was 50µl; and condition for the reaction was that GeneAmp PCR System 9600 (Perkin Elmer) was used and a cycle consisting of 98°C for 15 seconds and 68°C for 30 seconds was conducted 20 cycles. Each of the resulting PCR products was subcloned into pGEM-Teasy vector (manufactured by Promega) by TA cloning method, and the amplified fragments were cut out by digesting with KpnI and with BamHI and inserted into KpnI/BamHI site of pGL3-Basic Vector (manufactured by Promega). Base sequences of inserting regions of the four luciferase vectors, i.e. pGL3/De16324 (containing the positions 6324-6651 of the base sequence depicted in SEQ ID NO:4), pGL3/De16491 (containing the positions 6491-6651 of the same), pGL3/De16560 (containing the positions 6560-6651 of the same) and pGL3/De16578 (containing the positions 6578-6651 of the same) were determined, whereby the direction and the base sequence of the inserted fragment were found to be correct and then those plasmids were prepared in large quantities.

### 2) Luciferase assay

The following luciferase assay was carried out using five luciferase vectors, i.e. pGL3/1M prepared in Example 7 and pGL3/De16324, pGL3/De16491, pGL3/De16560 and pGL3/De16578 prepared in the above 1).

First, MC3T3-E1 derived from calvaria of mouse (supplied by Dr. Kumegawa, Department of Dentistry, Meikai University) and an undifferentiated mesenchymal cell line C3H10T1/2 derived from fetus of mouse (Institute of Physical and Chemical Research, Cell Bank) were plated in a 12-well cell culture plate so that cell numbers become 2 × 10⁵ cells/12 wells. Incubation was carried out using αMEM medium and BME medium (manufactured by Gibco BRL) respectively each containing 10% of fetal bovine serum (Gibco).

As a DNA mixture for introduction, a mixture comprising 0.5µg of each of the above luciferase vectors and 0.3µg of a β-galactosidase expression vector (PCH110 manufactured by Pharmacia) per well was prepared. To this DNA mixture was added pBluscribe M13+ plasmid (manufactured by Stratagene) so as to make the DNA amount in each well become 1µg in total and, at the time of 24 hours after plating of the cells, a gene transfection was carried out using LT-1 (Panvera).

After 4 hours from the gene transfection by the LT-1, the culture medium was changed for the above medium and incubation was conducted for two days. At the time of the completion of the incubation, the cells were lyzed in 150µl of Reporter Lysis Buffer (manufactured by Promega) and β-galactosidase activity was measured by a conventional method using 35µl of the above solution to determine the efficiency of introduction of DNA into the cells. Luciferase activity was detected by measuring the strength of luminescence during 10 seconds in a luminometer ML 3000 (Dynatech Laboratories) using 15µl of a cell lysate and a luciferase assay reagent (manufactured by Promega) as a substrate. The promoter activity of each vector was given by a relative value obtained by dividing the luciferase activity by β-galactosidase activity and defining the value of promoter-less pGL3-Basic as 1.

The result is shown in Fig. 5. In all of the cells, pGL3/De16324 and pGL3/De16491 showed a promoter activity which was the same as or a bit lower than pGL3/1M. However, in both pGL3/De16560 and pGL3/De16578, no promoter activity was detected. From the above, it was found that the sequence of the positions 6491-6560 of SEQ ID NO:4 played an important role in expression of the promoter activity of PEBP2αA (I) gene.

### Example 12.

### Construction of pGL3/+7, +45, +124 and 2M and Luciferase Assay

It was mentioned already in Example 6 that, in the positions 6652-7052 of the base sequence depicted in SEQ ID NO:4 (between the first and second methionines), there are many binding sequences for such as PEBP2 family and, in order to investigate how the nucleotide sequence between the first and second methionines affects the promoter activity of PEBP2αA (I) gene, the following experiments were carried out.

### 1) Construction of pGL3/+7, +45 and +124 and pGL3/2M

The three luciferase vectors, i.e. pGL3/+7, +45 and +124, were constructed using the genome DNA depicted in SEQ ID NO:4. The sequence of positions 4834-6665, 4834-6703 and 4834-6782 were amplified by PCR using a sense primer 2K3'B (5'-TAC-GGA-TCC-TAA-AGA-ATC-TTA-TGA-ACA-TGA-T-3' [SEQ ID NO:65]) consisting of the positions 4834-4855 of SEQ ID NO:4 having a BamHI-recognition sequence at 5'-terminal and an antisense primer B+7 (5'-GTG-GAT-CCG-GCG-AAT-GAA-GCA-TTC-ACA-CAA-3' [SEQ ID NO:82]) consisting of the positions 6665-6644 of SEQ ID NO:4 having a BamHI-recognition sequence at 5'-terminal, an antisense primer B+45 (5'-CCG-GAT-CCA-GTT-TGC-ACC-GCA-CTT-GTG-GTT-3' [SEQ ID NO:83]) consisting of the positions 6703-6682 of SEQ ID NO:4 having a BamHI-recognition sequence at 5'-terminal, and an antisense primer B+124 (5'-CTG-GAT-CCG-TTG-GTC-TCG-GTG-GCT-GGT-AGT-3' [SEQ ID NO:84]) consisting of the positions 6782-6761 of SEQ ID NO:4 having a BamHI-recognition sequence at 5'-terminal, respectively. Composition of the reaction mixture was 1 × PCR buffer, 0.2mM of each of dNTPs, 0.4µM of each primer, 200 ng of p26S3-1 (prepared in Example 6) and 1.25 units of Pfu DNA Polymerase (manufactured by Stratagene); volume of the reaction was 50µl; and condition of the reaction was that GeneAmp PCR System 9600 (Perkin Elmer) was used and a cycle comprising 94°C for 30 seconds, 57°C for 42 seconds and 72°C for 2 minutes was conducted 15 cycles. The resulting product was digested by a restriction enzyme BamHI and inserted into Bgl-II site of pGL3-Basic vector. The luciferase vectors constructed as such were named pGL3/+7, pGL3/+45 and pGL3/+124, respectively. After that, direction and total base sequence of the inserted fragment were determined by sequencing the inserted region including the cloning site, and then a large-scale production of each luciferase vector was carried out.

A luciferase vector pGL3/2M containing the sequence of the positions 4834-7052 of SEQ ID NO:4 was also prepared as follows. Thus, the sequence of the positions 4834-7052 of SEQ ID NO:4 was amplified by PCR using a sense primer 2K3'B (5'-TAC-GGA-TCC-TAA-AGA-ATC-TTA-TGA-ACA-TGA-T-3') (SEQ ID NO:65) consisting of the positions 4834-4855 of SEQ ID NO:4 having a BamHI-recognition sequence at 5'-terminal and an antisense primer 2M5'B (5'-TAC-GGA-TCC-AGT-CCC-TCC-TTT-TTT-TTC-CAG-ATA-G-3') (SEQ ID NO:85) consisting of the positions 7052-7027 of SEQ ID NO:4 having a BamHI-recognition sequence at 5'-terminal. Composition of the reaction mixture was 1 × PCR Buffer, 0.2 mM of each of dNTPs, 0.2µM of each primer, 10 ng of pP6S3-1 (prepared in Example 6) and 2 units of Amplitaq DNA polymerase (manufactured by ABI); volume for the reaction was 50µl; and condition for the reaction was that GeneAmp PCR System 9600 (Perkin Elmer) was used and a cycle consisting of 94°C for 30 seconds, 60°C for 30 seconds and 72°C for 30 seconds was carried out 15 cycles. The resulting product was digested with a restriction enzyme BamHI and inserted into BglII site of pGL3-Basic vector. The luciferase vector constructed as such was named pGL3/2M. After that, direction and total base sequence of the fragment were confirmed by sequencing the insert region including the cloning site, and a large-scale production was carried out.

### 2) Luciferase assay

A luciferase assay was carried out in the same manner as in Example 11 using five luciferase vectors, i.e., pGL3/1M prepared in Example 7 and pGL3/+7, pGL3/+45, pGL3/+124 and pGL3/2M prepared in the above 1). The cells used were C3H10T1/2 (Institute of Physical and Chemical Research, Cell Bank) and UMR-106 (obtained from Dainippon Pharmaceutical). The result is shown in Fig. 6. When the luciferase activity of pGL3/1M was used as a standard, the activity increased as the sequence became longer from pGL3/+7 to pGL3/+45 in all of the cells, which showed that there was an enhancer which increased the promoter activity in the sequence of the positions 6652-6703 of SEQ ID NO:4. As the sequence became much longer so that pGL3/+124 and pGL3/2M, the activity decreased, which showed that there was a silencer which reduced the promoter activity in the sequence of the positions 6704-7052 of SEQ ID NO:4.

Incidentally, the degree of increase and decrease of the above activity was greater in the case of UMR-106 which is a osteoblast-like cell than in the case of C3H10T1/2 which is an undifferentiated mesenchymal cell. This suggested that a factor which uniquely exists in the cells of bone lineage binds to the above enhancer or silencer, whereupon the increase or decrease of the activity takes place.

### Example 13.

### Investigation of the Effect of Various Transcription Factors on the Promoter Activity of PEBP2αA (I) Gene

Effect of PEBP2αA and PEBP2β2 which are the factors belonging to PEBP2 family, SOX9 which is a factor belonging to Sry family, and GATA3 which is a factor belonging to GATA family on the promoter activity of pGL3/1M and pGL3/2M was investigated.

First, 1) mouse PEBP2αA cDNA (Proc. Natl. Acad. Sci. USA, 90, 6859-6863 (1993)), 2) mouse PEBP2β2 cDNA (Virology, 194, 314-331 (1990)), 3) mouse SOX9 cDNA (Nature, 372, 525-530 (1994)) and 4) mouse GATA3 cDNA (Mol. Cell. Biol., 11, 2778-2784 (1991)) were cloned by PCR after preparing appropriate primers based upon the previously-reported sequences. Then, expression vectors were constructed by ligating to pSG5 (manufactured by Stratagene) for mouse PEBP2αA cDNA, to pcDNA3.1(+) (manufactured by Invitrogen) for mouse PEBP2β 2cDNA and mouse GATA 3 cDNA, and to pcDNA3.1/Hygro(+) (manufactured by Invitrogen) for mouse SOX9 cDNA.

A luciferase assay was carried out in a 12-well cell culture plate using a cell line COS1 derived from kidney of monkey (obtained from Dainippon Pharmaceutical), MC3T3-E1 derived from calvaria of mouse (Institute of Physical and Chemical Research, Cell Bank) and an undifferentiated mesenchymal cell line of mouse C3H10T1/2 (Institute of Physical and Chemical Research, Cell Bank). COS1 was incubated in DMEM medium (Gibco BRL) containing 10% of fetal bovine serum (manufactured by Gibco BRL), MC3T3-E1 was incubated in αMEM medium (Gibco BRL), containing 10% of fetal bovine serum (manufactured by Gibco BRL), and C3H10T1/2 was incubated in BME medium (Gibco BRL) containing 10% of fetal bovine serum (manufactured by Gibco BRL).

As a DNA mixture for the introduction, 0.2µg of pGL3/1M or pGL3/2M and 0.3µg of β-galactosidase expression vector (PCH110 manufactured by Pharmacia) were prepared per well. After that, each 0.1µg of various transcription factor expression vectors prepared above was, or was not, added thereto. To such a DNA mixture was added pBluscribeM13+ plasmid (manufactured by Stratagene) so that the DNA amount for each well becomes 1.0µg in total and, at the time that each cell became 40-60% confluent, the above DNA mixture was transfected using LT-1 (manufactured by Panvera). In the case of PEBP2, however, 0.1µg of an expression vector for β subunit (PEBP2β2) was added simultaneously with the addition of 0.1µg of α subunit (PEBP2αA). Gene introduction by LT-1 was performed for 4 hours and, after changing the medium, incubation was carried out for 44 hours. After that, the cells were lyzed in 150µl or 180µl of Reporter Lysis Buffer (manufactured by Promega) and β-galactosidase activity was measured by a conventional method using 35µl of the resulting lysate to determine the efficiency of the introduction of DNA into the cells. A luciferase activity was detected by measuring the strength of luminescence within 10 seconds by a luminometer ML3000 (Dynatech Laboratories) using 15µl of the cell lysate and a luciferase assay reagent (Promega) as a substrate. Promoter activity of each vector was given using a relative value obtained by dividing a luciferase activity by a β-galactosidase activity and defining the value of promoter-less pGL3basic vector as 1.

The result is shown in Table 8. In the table, 1M and 2M stand for the result of introduction of pGL3/1M and pGL3/2M, respectively, and P, S and G stand for the result of introduction of expression vectors of PEBP2 (α and β), SOX9 and GATA3, respectively. It was found that, when those factors were expressed in the cell, promoter activity of pGL3/1M or pGL3/2M was regulated. In the sequence of the positions 4834-6651 of SEQ ID NO:4 in pGL3/1M and the sequence of the positions 4834-7052 of SEQ ID NO:4 in pGL3/2M, there were many binding sequences for PEBP2 family, Sry family and GATA family and similar sequences thereto as shown in Table 1. Accordingly, it is believed that the promoter activity was regulated due to the fact that such transcription factors expressed in the cells were bound to the binding sequences on pGL3/1M and 2M.

**Table 8**

| Cell | Promoter | Vector | Fold |
|---|---|---|---|
| C3H10T1/2 | 1M | P | 3.2 |
| | | G | 0.68 |
| | 2M | P | 3.6 |
| | | G | 0.48 |
| COS1 | 1M | P | 4.8 |
| | | S | 1.9 |
| | | G | 0.68 |
| | 2M | P | 2.9 |
| | | G | 0.42 |
| MC3T3-E1 | 1M | P | 2 |
| | | S | 1.9 |
| | | G | 0.69 |
| | | P/S | 2.6 |
| | 2M | P | 1.9 |
| | | S | 1.5 |
| | | G | 0.75 |
| | | P/S | 3.5 |

### Example 14.

### Investigation of Promoter Activity of PEBP2αA (I) Gene in Various Cells

Promoter activity of PEBP2αA (I) gene in various cells was compared.

A luciferase assay was carried out in the same manner as in Example 11 using pGL3/1M and pGL3/2M as the luciferase vectors for introduction and UMR-106 cell (Dainippon Pharmaceutical), C3H10T1/2 (Institute of Physical and Chemical Research, Cell Bank), COS-1 (Dainippon Pharmaceutical) and HepG2 (Dainippon Pharmaceutical) as the cells.

The result is shown in Fig. 7. In all of the cases of using pGL3/1M and pGL3/2M, the strongest activity was observed in UMR-106 cell which was an osteoblast-like cell line and the second strongest activity was observed in C3H10T1/2 which was an undifferentiated cell line having a property of being differentiated to osteoblasts. Accordingly, it was believed that an expression regulatory region of PEBP2αA (I) gene uniquely functions in an osteoblast lineage.

### Example 15

### Analysis of PEBP2αA Gene Expression by Northern Hybridization

### 1) Extraction of RNA

AGPC method (Anal. Biochem., 162, 156-159 (1987)) was used for extraction of total RNA from cells and fetuses, while improved AGPC method (Biotechniques, 8, 148-149) was used for extraction from bone tissues. Purification of mRNA from total RNA was carried out using mRNA Purification Kit (manufactured by Pharmacia Biotech) or Dynabeads Oligo (dT)25 (manufactured by Dynal) according to the protocol attached thereto.

### 2) Northern hybridization

mRNA (2µg) extracted by the above method was subjected to an electrophoresis using 1.1M formaldehyde-1% agarose gel and transferred to Hybond N+ (manufactured by Amersham) by means of a capillary action by 20XSSC. The RNA-transferred membrane was subjected to UV cross-linking and then subjected to northern hybridization as follows. Thus, as to a probe specific to PEBP2αA (I) gene, a fragment of about 300 bp (SEQ ID NO:3) amplified by two primers, i.e., a primer K-UTR for 5'-UTR immediately upstream of the first methionine (K-UTR: 5'-AAAGC TTTTG CTTTT TTGGA TTGTG TG-3' [SEQ ID NO: 70]) and a primer K-AS (K-AS: 5'-CCAAA AGAAG CTTTG CTGAC ACGG-3') [SEQ ID NO:71]) corresponding to the immediate upstream of the common region coding for the protein encoded by the previously-reported PEBP2αA gene (Genbank Accession Number: D14636) was used. A probe which was specific to PEP2αA (II) gene was prepared by amplifying by means of PCR of the positions 319-1015 of SEQ ID NO:2 prepared in Example 2. All of those probes were labeled with ³²P-dCTP by BcaBEST Labeling Kit (manufactured by Takara Shuzo). After prehybridization in a solution of 50% formamide, 5 × SSPE, 5 × Denhart's solution, 1 mg/ml of salmon sperm DNA and 0.1% of SDS at 39°C for more than 4 hours, a hybridization was carried out in 50% formamide, 5 × SSPE, 1 × Denhart's solution, 0.2 mg/ml of salmon sperm DNA and 1 × 10⁶ cpm/ml of ³²P-labeled probe at 39°C for 20 hours. Washing was carried out in a solution of 2 × SSPE, 0.03% of NaPPi and 0.1% of SDS at room temperature for 10 minutes and then in a solution of 1 × SSPE, 0.03% of NaPPi and 0.1% of SDS at 37°C for 20 minutes. After washing, autoradiography was carried out using BAS2000 Image Analyzer. In addition, a probe for the base sequence common to PEBP2αA (I) and (II) genes (a region coding for protein) corresponding to nucleotides of 1293-1688 of Genbank Accession Number: D14636 was prepared, and the similar experiment as above was carried out using the probe.

The result are shown in Fig. 1 and Fig. 2. Expression of mRNA corresponding to PEBP2αA (I) gene was observed in bone tissues (B in Fig. 2) and, clear signals were detected in osteoblast-like cell lines such as ROS17/2.8, UMR-106 and MC3T3-E1 (B in Fig. 1), while in B and T cell lines such as EL 4 and BW5147, no expression was observed (B in Fig. 2). It was also found that the size of the mRNA in bone tissues was around 6 kb. On the other hand, expression of the mRNA corresponding to PEBP2αA (II) gene was observed in all cells investigated (C in Fig. 2). Size of this mRNA was believed to be around 7 kb. The reason why no clear signal was detected in mRNA derived from bone tissues as compared with EL4 and BW 5147 is probably due to the fact that, in EL4 and BW 5147, mRNA corresponding to the sequence registered in Genbank (Accession Number: D14636) was expressed whereby a strong hybridization with the probe took place, while in the bone tissues, mRNA having an start site for the transcription near the position 991 of the base sequence depicted in SEQ ID NO:2 was expressed (cf. Example 2), and therefore, the expressed RNA contained only short region capable of hybridizing the probe used.

From the above result, it was found that expression of the mRNA corresponding to PEBP2αA (I) gene was strong in bone tissues and in the cells more differentiated to osteoblasts. On the other hand, expression of the mRNA corresponding to PEBP2αA (II) gene showed no particular specificity to bone tissues but was generally observed in fibroblastic cell line, B cell line, T cell line and thymus tissue where expression of PEBP2αA gene was reported (Mol. Cell. Biol., 15, 1662-1670 (1995); Proc. Natl. Acad. Sci. USA, 90, 6859-6863 (1993)). However, as mentioned previously, it is believed that PEBP2αA mRNA which is usually expressed in cell lines corresponds to the sequence registered as a mouse PEBP2αA cDNA (Accession Number: D14636) and there is a possibility that it is transcribed from a different promoter of mRNA expressed in bone tissue.

### Example 16

### Construction of High Throughput Screening System

### 1) Preparation of Stable Cells

C3H10T1/2 cells derived from fetus of mouse were plated in a culture dish having a diameter of 100 mm so that 40-70% confluent may be obtained. After one day, 1µg of pSV2neo (manufactured by BRL) and 24µg of luciferase vector prepared either in Example 1 or 2 (thus, 25µg in total) were introduced either by a calcium phosphate method or by LT-1 (manufactured by Panvera). Two days after the gene transfection, the cells were plated on 3 culture dishes each having a diameter of 100 mm in a cell density of 1/100 and, at the same time, selection by an antibiotic G418 (500µg/ml) was initiated. The grown colonies were once again plated in a cell density of 1/100 to form colonies again. The grown colonies were treated with trypsin using a colony ring and transferred to a 24-well culture plate.

### 2) Selection of the cells suitable for screening

When the cells transferred to a 24-well culture dish grew, part of them was plated in a 96-well culture dish at the stage of passage and, after one day, BMP-4 was added. At that time, nothing was added to some wells. Then, after 24 hours, 48 hours and 96 hours, the culture medium was sucked and washed with PBS(-), and 20µl of a solution for cell lysis (manufactured by Promega) were added to lyze the cells. A luciferase activity was detected by measuring the strength of luminescence within 10 seconds by a luminometer ML3000 (Dynatech Laboratories) using Luciferase Assay Substrate (manufactured by Promega) as s substrate.

Table 9 shows examples of selection of the cells which showed high activity due to addition of BMP-4, in other words, the cells by which the action of test samples to the promoter activity is more easily detectable. Table 9 shows the relative luciferase activity of the BMP-added group when the value where no BMP was added was defined as 1 (n = 2). The luciferase vector used for the introduction was pGL3/1811. In all of the clones K23, K27 and K24 listed in Table 9, the activity increased to 1.2- to 1.8-fold by the treatment with BMP for 24 hours and the activity increased to 1.6- to 2.3-fold by the treatment with BMP for 96 hours. Thus, they were the cells having very good responsibility. Those cells were subjected to an enlarged culture and stocked in liquid nitrogen.

**Table 9**

| | Clones: K23 | K24 | K27 |
|---|---|---|---|
| Treating Time | | | |
| 6 hours | 1 | 1 | 1 |
| 24 hours | 1.8 | 1.2 | 1.2 |
| 48 hours | 2.3 | 1.5 | 1.5 |
| 96 hours | 2.3 | 2.6 | 1.6 |

Table 10 shows the result of selection of the cells to be used for screening, in which pGL3/1M was used as a luciferase vector, and C3H10T1/2 and MC3T3-E1 were used as the cells, and the treatment with BMP-4 or all-trans retinoic acid (final concentration: 1µM) was carried out for two days in the same manner as above. Cells showing good responsibility to BMP or retinoic acid were also obtained. Those cells were subjected to an enlarged culture and stocked in liquid nitrogen as well.

**Table 10**

| Cell line | C3H10T1/2 | | | MC3T3-E1 | |
|---|---|---|---|---|---|
| Name of clone | 17-7 | 08-2 | 08-16 | 11-13 | 11-15 |
| No stimulation | 1 | 1 | 1 | 1 | 1 |
| BMP | 5.8 | 1.2 | 1.6 | 1.2 | 1.1 |
| Retinoic acid | 1.3 | 2.2 | 1.9 | 1.7 | 2.1 |

### 3) High throughput screening

The cells prepared in the above 2) were plated in a 96-well culture plate to make 400,000 cells/100µl/well and incubated for 24 hours. The culture medium used was Eagle's basal medium (BME) (manufactured by Gibco) to which 10% fetal bovine serum was added in the case of C3H10T1/2. To each well were added 25µl of a solution of a compound to be tested (10µg/ml) (final concentration: 2µg/ml) and incubation was continued for 24 hours. After completion of the incubation, the supernatant was sucked, a substrate for luciferase (LucLite manufactured by Packard) was added thereto, and a quantitative determination of luminescence was performed.

### INDUSTRIAL APPLICABILITY

The present invention provides for a DNA which participates in the regulation of expression of PEBP2αA gene; a recombinant plasmid wherein the DNA is ligated to a reporter gene; a cell which is transformed with the plasmid; a method for screening an expression regulator for PEBP2αA gene using the transformed cell; and an expression regulator for PEBP2αA gene obtained by the screening method. Among the expression regulators for the PEBP2αA, an expression enhancer for PEBP2αA gene is believed to increase the expression amount of PEBP2αA whereby osteogenesis is promoted and, therefore, it is useful as an osteogenesis stimulator.

## Claims

1. A DNA which participates in the regulation of expression of a PEBP2αA gene wherein said DNA
(a) comprises all or part of the base sequence depicted in any one of SEQ ID NO: 1, SEQ ID NO: 4 or SEQ ID NO 5;
(b) comprises all or part of the sequence at positions 4834-7052 of the base sequence depicted in SEQ ID NO: 4;
(c) comprises all or part of the sequence at positions 319-1015 in the base sequence depicted in SEQ ID NO: 2;
(d) comprises the DNA of (a) or (b) wherein said DNA comprises at least the sequence at positions 1693-1762 in the base sequence depicted in SEQ ID NO: 1 or the corresponding sequence at positions 6491-6560 in the base sequence depicted in SEQ ID NO: 4;
(e) comprises the DNA of (a) or (b) wherein said DNA comprises at least the sequence at positions 1113-1811 in the base sequence depicted in SEQ ID NO: 1 or the corresponding sequence at positions 5911-6609 in the base sequence depicted in SEQ ID NO: 4;
(f) comprises the DNA of (a) or (b) wherein said DNA comprises any one of the binding sequences of the PEBP2 family, the binding sequences of the Sry family, the binding sequences of the GATA family or sequences similar to those binding sequences, as listed in the following Table:
| Positions in SEQ ID NO:4 | Positions in SEQ ID NO:1 |
|---|---|
| | |
| Binding sequences of the PEBP2 family | |
|---|---|
| positions 5627-5633 | positions 829-835 |
| positions 6307-6313 | positions 1509-1515 |
| positions 6571-6577 | positions 1773-1779 |
| positions 6674-6680 | - |
| positions 6682-6688 | - |
| positions 6801-6807 | - |
| Binding sequences of the Sry family | |
|---|---|
| positions 5069-5075 | positions 271-277 |
| positions 5424-5435 | positions 626-637 |
| positions 5600-5606 | positions 802-808 |
| positions 5644-5650 | positions 846-852 |
| positions 5756-5762 | positions 958-964 |
| positions 5925-5931 | positions 1127-1133 |
| positions 5936-5942 | positions 1138-1144 |
| positions 6630-6636 | positions 1832-1838 |
| positions 6670-6676 | - |
| positions 6853-6859 | - |
| positions 6858-6864 | - |
| Binding sequences of the GATA family | |
|---|---|
| positions 5014-5023 | positions 216-225 |
| positions 5050-5059 | positions 252-261 |
| positions 5093-5106 | positions 295-308 |
| positions 5162-5171 | positions 364-373 |
| positions 5288-5297 | positions 490-499 |
| positions 5578-5588 | positions 780-790 |
| positions 5676-5685 | positions 878-887 |
| positions 5790-5799 | positions 992-1001 |
| positions 5836-5845 | positions 1038-1047 |
| positions 6484-6492 | positions 1686-1694 |
| positions 6576-6585 | positions 1778-1787 |
| positions 7024-7036 | - |
(g) contains a deletion, a substitution, and/or an addition of one or more bases in the DNA of any one of (a) to (f); or
(h) hybridizes under stringent conditions with the DNA of any one of (a) to (g).

2. A plasmid in which a reporter gene or a promoter gene and a reporter gene are ligated to the DNA described in claim 1.

3. A plasmid in which a reporter gene is ligated to a DNA selected from the group consisting of:
(a) a DNA comprising all or part of the base sequence depicted in SEQ ID NO:2 and participating in the regulation of the expression of a PEBP2αA gene;
(b) a DNA comprising all or part of the sequence of positions 319-1015 in the base sequence depicted in SEQ ID NO: 2 and participating in the regulation of expression of a PEBP2αA gene; and
(c) a DNA which hybridizes with the DNA of the above (a) or (b) under stringent conditions or the DNA according to the above (a) or (b) which contains a deletion, a substitution, and/or an addition of one or more bases, said DNA participating in the regulation of expression of a PEBP2αA gene.

4. The plasmid of claim 2 or 3, wherein said reporter gene is a luciferase, a CAT, a GH or an ALP gene.

5. A transformed cell obtained by introducing the plasmid described in claim 2, 3 or 4.

6. A method of screening for an expression regulator for a PEBP2αA gene, characterized by adding a substance to be tested to the transformed cell of claim 5 and detecting whether the substance affects the DNA of claim 1.

7. An expression regulator for a PEBP2αA gene obtained by the screening method of claim 6.

8. An expression enhancer for a PEBP2αA gene obtained by the screening method of claim 6.

9. A method of screening for an expression regulator for a PEBP2αA gene, characterized by adding a substance to be tested to the transformed cell of claim 5 and detecting whether the substance affects:
(a) a DNA comprising all or part of the base sequence depicted in SEQ ID NO:2 and participating in the regulation of the expression of a PEBP2αA gene;
(b) a DNA comprising all or part of the sequence at positions 319-1015 in the base sequence depicted in SEQ ID NO:2 and participating in the regulation of the expression of a PEBP2αA gene; and/or
(c) a DNA which hybridizes with the DNA of the above (a) or (b) under stringent conditions or the DNA according to the above (a) or (b) which contains a deletion, a substitution, and/or an addition of one or more bases, said DNA participating in the regulation of the expression of a PEBP2αA gene.

10. A pharmaceutical composition comprising the DNA of claim 1.

11. A pharmaceutical composition comprising an expression regulator of claim 7.

12. The use of a DNA of claim 1 for the preparation of a pharmaceutical composition for gene therapy.

13. The use of an expression regulator of claim 7 for the preparation of a pharmaceutical composition for treating bone diseases.
